Europäisches Patentamt

European Patent Office

Office européen des brevets

(19)

(11) **EP 1 059 947 B1**

(12) **EUROPÄISCHE PATENTSCHRIFT**

(45) Veröffentlichungstag und Bekanntmachung des Hinweises auf die Patenterteilung:
**29.09.2004 Patentblatt 2004/40**

(21) Anmeldenummer: **99913215.2**

(22) Anmeldetag: **03.03.1999**

(51) Int Cl.⁷: **A61L 15/60**, C08J 9/30

(86) Internationale Anmeldenummer:
**PCT/EP1999/001363**

(87) Internationale Veröffentlichungsnummer:
**WO 1999/044648 (10.09.1999 Gazette 1999/36)**

(54) **WASSERABSORBIERENDE, SCHAUMFÖRMIGE, VERNETZTE POLYMERISATE, VERFAHREN ZU IHRER HERSTELLUNG UND IHRE VERWENDUNG**

WATER-ABSORBING, CROSS-LINKED POLYMERIZATES IN THE FORM OF A FOAM, A METHOD FOR THE PRODUCTION THEREOF, AND THEIR USE

POLYMERISATS ABSORBANT L'EAU, SOUS FORME DE MOUSSE, RETICULES, LEUR PROCEDE DE PRODUCTION ET LEUR UTILISATION

(84) Benannte Vertragsstaaten:
**DE ES FR GB IT SE**

(30) Priorität: **05.03.1998 DE 19809540**

(43) Veröffentlichungstag der Anmeldung:
**20.12.2000 Patentblatt 2000/51**

(73) Patentinhaber: **BASF AKTIENGESELLSCHAFT 67056 Ludwigshafen (DE)**

(72) Erfinder:
• **HÄHNLE, Hans-Joachim D-67435 Neustadt (DE)**
• **SCHRÖDER, Ulrich D-67227 Frankenthal (DE)**
• **BECK, Martin D-67251 Freinsheim (DE)**
• **HEIDER, Wolfgang D-67434 Neustadt (DE)**
• **SCHORNICK, Gunnar D-67271 Neuleiningen (DE)**
• **ANSTOCK, Thomas D-67256 Weisenheim (DE)**

(56) Entgegenhaltungen:
**EP-A- 0 712 659          DE-A- 19 607 529**

• **CHEMICAL ABSTRACTS, vol. 125, no. 4, 22. Juli 1996 (1996-07-22) Columbus, Ohio, US; abstract no. 35562, HARADA, NOBUYUKI ET AL: "Manufacture of water-absorbing sheets with easy handling and excellent flexibility and water absorption properties and their water-absorbing products" XP002110926 & JP 08 073507 A (NIPPON CATALYTIC CHEM IND, JAPAN) 19. März 1996 (1996-03-19)**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft wasserabsorbierende, schaumförmige, vernetzte Polymerisate, Verfahren zur ihrer Herstellung und ihre Verwendung in Sanitärartikeln, die zur Absorption von Körperflüssigkeiten und in Verbandmaterial zur Abdeckung von Wunden eingesetzt werden.

Wasserabsorbierende, vernetzte Polymerisate werden als Superabsorber oder superabsorbierende Polymere bezeichnet, weil sie in der Lage sind, ein Vielfaches ihres Eigengewichtes an wäßrigen Flüssigkeiten unter Ausbildung von Hydrogelen aufzunehmen. In der Praxis werden Superabsorber beispielsweise in Windeln zur Absorption von Urin eingesetzt. Die Superabsorber haben die Eigenschaft, die absorbierte Flüssigkeit auch unter mechanischer Belastung zurückzuhalten.

Um die anwendungstechnischen Eigenschaften von Superabsorbern zu variieren, sind zwei unterschiedliche Typen von Schäumen bekannt, (1) Mischungen, die Superabsorber in einer geschäumten Matrix enthalten, und (2) Schäume, die aus einem superabsorbierenden Material bestehen.

Ein unter die Kategorie (1) fallender Schaum wird beispielsweise aus einer Mischung hergestellt, die einerseits Komponenten für die Bildung eines Polyurethanschaums und andererseits polymerisierbare Monomere, einen Vernetzer und einen Polymerisationsinitiator zur Herstellung eines Superabsorbers enthält. Aus einer solchen Mischung wird in einer Polykondensationsreaktion aus den Polyurethankomponenten der Schaum gebildet, der den durch Polymerisation der Monomeren entstehenden Superabsorber in Form eines interpenetrierenden Netzwerkes enthält, vgl. US-A-4 725 628, US-A-4 725 629 und US-A-4 731 391.

Aus der US-A-4 985 467 ist ein Polyurethanschaum bekannt, der einen Superabsorber chemisch gebunden enthält. Außerdem sind Kombinationen von Latex-Schäumen bekannt, in die nach dem Schäumprozeß superabsorbierende, feinteilige Materialien eingearbeitet werden, vgl. EP-A-427 219 und US-A-4 990 541.

Zu der Kategorie (2) von Schäumen gehören beispielsweise solche Produkte, die dadurch erhalten werden, daß man einen vorgefertigten Superabsorber in einem Extruder mit einer Polyhydroxyverbindung und einem Treibmittel bei erhöhter Temperatur mischt. Beim Auspressen der Mischung aus dem Extruder bildet sich der Schaum. Verfahren dieser Art sind beispielsweise in der US-A-4 394 930, US-A-4 415 388 und GB-A-2 136 813 beschrieben.

Aus US-A-4 529 739 und US-A-4 649 154 sind Verfahren zur Herstellung von Schäumen bekannt, wobei man ein wasserquellbares, COOH-Gruppen tragendes Material mit einem Treibmittel aufschäumt, das in einer Neutralisierungsreaktion mit den COOH-Gruppen des Polymeren das Treibgas freisetzt.

Gemäß den Angaben in der WO-A-94/22502 werden superabsorbierende Schäume auf Basis von vernetzten, teilweise neutralisierten Polycarboxylaten dadurch hergestellt, daß man eine Monomermischung mit einem in Wasser unlöslichen Treibmittel schäumt, das einen Siedepunkt unterhalb von 50°C hat, und den Schaum praktisch gleichzeitig mit dem Schäumen auspolymerisiert.

Aus der EP-A-04 21 264 ist die Herstellung schaumartiger Superabsorber bekannt, wobei man eine wäßrige Monomermischung, die eine Ölphase emulgiert enthält, polymerisiert. Das Öl wirkt hierbei als Platzhalter für die späteren Poren des Schaums und wird nach beendeter Polymerisation beim Trocknen des schaumförmigen Materials durch Verdampfen entfernt.

Aus der WO-A-88/09801 ist bekannt, daß man hydrophile Polymere, z.B. Polynatriumacrylat in Gegenwart von Vernetzern wie Polyepoxiden und Treibmitteln durch Erwärmen zu einem schaumförmigen Superabsorber verarbeiten kann.

Aus der DE-A-196 07 529 ist ein Absorberelement aus einem Verbundmaterial bekannt, bei dem Elemente aus einem superabsorbierenden Schaum rasterförmig in solchen Abständen auf einem Träger angeordnet sind, daß sich die Elemente in gequollenem Zustand an ihren Umfängen berühren. Der superabsorbierende Schaum wird durch (I) Schäumen einer polymerisierbaren wäßrigen Mischung, die als wesentliche Komponenten Säuregruppen enthaltende ethylenisch ungesättigte Monomere, Vernetzer und Tenside enthält, nach der Schlagschaummethode und (II) Polymerisieren der geschäumten Mischung unter Bildung eines schaumförmigen Hydrogels hergestellt. Der Wassergehalt des Schaustoffs wird gegebenenfalls auf 1 bis 60 Gew.-% eingestellt.

Aus Chemical Abstracts, Vol. 125, No. 4, (1996-07-22), Abstract No. 35562, sind wasserabsorbierende flächige Gebilde bekannt, die durch Polymerisieren von beispielsweise Acrylsäure und Alkanolaminsalzen in Gegenwart von Vernetzern in dünner Schicht hergestellt werden. Die so hergestellten Schäume werden als wasserabsorbierende Komponente in Windeln und in Inkontinenzartikeln verwendet.

Zur Herstellung schaumförmiger Superabsorber ist außerdem eine Arbeitsweise bekannt, bei der man Carbonate, Hydrogencarbonate oder Kohlendioxid als Treibmittel zu einer Mischung aus Carboxylgruppen tragenden Monomeren, Vernetzungsmittel und Polymerisationsinitiator zusetzt, wobei zeitgleich mit der Zugabe des Treibmittels oder kurz darauf die Polymerisation der Monomeren gestartet wird. Der Superabsorber erhält durch das bei der Neutralisationsreaktion gebildete Kohlendioxid eine Schaumstruktur, vgl. EP-A-2 954 438 und US-A-4 808 637. Nach dem aus der WO-A-95/02002 bekannten Verfahren wird ein schaumförmiger Superabsorber im Anschluß an die Herstellung mit einer und mehreren zur nachträglichen Oberflächenvernetzung reaktionsfähigen Verbindungen versetzt und auf eine Temperatur von 100 bis 300°C erhitzt.

Bei den oben beschriebenen Verfahren zur Herstellung von superabsorbierenden Schäumen erfolgt die Bildung des Schaums und die Polymerisation entweder synchron oder nur unwesentlich zeitlich versetzt. Die noch nicht auspolymerisierten Schäume haben nur eine geringe Standzeit, meistens beträgt sie nur wenige Minuten. Nachteilig bei den oben angegebenen Verfahren ist beispielsweise der Einsatz größerer Treibmittelmengen, insbesondere der Einsatz von FCKW im Fall der WO-A-94/22502.

Aus der DE-A-19607551 sind wasserabsorbierende, schaumförmige, vernetzte Polymerisate bekannt, die erhältlich sind durch

(I) Schäumen einer polymerisierbaren wäßrigen Mischung, die

(a) Säuregruppen enthaltende monoethylenisch ungesättigte Monomere, die zu mindestens 50 Mol-% neutralisiert sind,

(b) gegebenenfalls andere monoethylenisch ungesättigte Monomere,

(c) Vernetzer,

(d) Initiatoren,

(e) 0,1 bis 20 Gew.-% mindestens eines Tensids,

(f) gegebenenfalls mindestens einen Lösevermittler und

(g) gegebenenfalls Verdicker, Schaumstabilisatoren, Polymerisationsregler, Füllstoffe und/oder Zellkeimbildner

enthält, wobei das Schäumen durch Dispergieren von feinen Blasen eines gegenüber Radikalen inerten Gases in die polymerisierbare wäßrige Mischung erfolgt, und

(II) Polymerisieren der geschäumten Mischung unter Bildung eines schaumförmigen Hydrogels und gegebenenfalls Einstellen des Wassergehalts des Polymerisats auf 1 bis 60 Gew.-%.

Nach der Lehre der DE-A-19540951 wird der Wassergehalt des schaumförmigen Hydrogels auf 1 bis 45 Gew.-% eingestellt. Die so erhältlichen schaumförmigen Polymeren werden in Sanitärartikeln, z.B. zur Absorption von Körperflüssigkeiten verwendet.

In der JP-A-08073507 werden weiche und flexible Superabsorberfilme schrieben, die dadurch herstellt werden, daß eine wäßrige Acrylatlösung, die zum Teil durch ein Alkanolamin neutralisiert ist, in Gegenwart eines Vernetzungsmittels polymerisiert wird. Die erhaltenen Filme werden zwar als weich und flexibel beschrieben, es werden jedoch keine Angaben darüber gemacht, unter welchen klimatischen Bedingungen die Flexibilität erhalten bleibt. Insbesondere sind keine Angaben für erhöhte bzw. erniedrigte Temperaturen verfügbar. Außerdem weisen diese Produkte mehrere gravierende Nachteile auf. So ist ihre Aufnahmegeschwindigkeit für die Anwendung in Hygieneartikeln völlig unzureichend und auch ihre Aufnahmekapazität ist noch verbesserungsbedürftig. Fernerhin zeigen die Filme eine die Handhabung stark einschränkende, ausgeprägte Klebrigkeit.

Superabsorberschäume auf der Basis von Säuregruppen-tragenden, vernetzten Polymerisaten, die z.B. entsprechend den obengenannten Literaturstellen DE-A-19540951, DE-A-19607551 oder W-A-094/22502 hergestellt werden, können durch die Einstellung eines definierten Feuchtegehaltes von ca. 25 % weich und flexibel erhalten werden. Selbst wenn zusätzliche Flexibilisierungsmaßnahmen, wie in den obigen Patenten beschrieben, ergriffen werden, ist dennoch in der Regel ein Feuchtegehalt von mindestens 20 % notwendig. Die Flexibilisierung durch einen so hohen Wassergehalt bringt den Nachteil mit sich, daß sie bei geringen Luftfeuchten nicht stabil ist. Bei einer relativen Luftfeuchte unter 50 % beginnt der Schaum auszutrocknen und verliert so zunehmend seine Flexibilität. Ebenso nimmt die Flexibilität des Schaums bei Temperaturen unterhalb von 5°C rasch ab.

Der Verlust der Flexibilität ist zwar reversibel, d.h. bei ansteigender Temperatur bzw. bei Erhöhung der Luftfeuchte werden die Schäume wieder weich und flexibel, aber für eine Anwendung stellt dies dennoch eine gravierende Einschränkung dar, z.B. wenn ein Hygieneartikel in sehr trockener Wohnungsluft gelagert wird. Fernerhin fühlen sich die erhaltenen Schaumschichten feucht an (feuchter "Griff"), was für die Anwendung in Hygieneartikeln ebenfalls als nachteilig angesehen wird.

Der Erfindung liegt die Aufgabe zugrunde, einen superabsorbierenden Schaum zur Verfügung zu stellen, der unter anwendungsrelevanten Bedingungen, z.B. bei Temperaturen zwischen ·15°C und +50°C und einer relativen Luftfeuchte von 20 bis 95 %, beständig flexibel ist und gleichzeitig die übrigen geschilderten Nachteile überwindet.

Die Aufgabe wird erfindungsgemäß gelöst mit wasserabsorbierenden, schaumförmigen, vernetzten Polymerisaten, die erhältlich sind durch

(I) Schäumen einer polymerisierbaren wäßrigen Mischung, die

(a) Säuregruppen enthaltende monoethylenisch ungesättigte Monomere, die gegebenenfalls neutralisiert sind,

(b) gegebenenfalls andere monoethylenisch ungesättigte Monomere,

(c) Vernetzer,

(d) Initiatoren,

(e) 0,1 bis 20 Gew.-% mindestens eines Tensids,

(f) gegebenenfalls mindestens einen Lösevermittler und

(g) gegebenenfalls Verdicker, Schaumstabilisatoren, Polymerisationsregler, Füllstoffe und/oder Zellkeimbilder

enthält, wobei das Schäumen durch Dispergieren von feinen Blasen eines gegenüber Radikalen inerten Gases in die polymerisierbare wäßrige Mischung erfolgt, und

(II) Polymerisieren der geschäumten Mischung unter Bildung eines schaumförmigen Hydrogels und gegebenenfalls Einstellen des Wassergehalts des schaumförmigen Polymerisats auf 1 bis 60 Gew.-%, wenn mindestens 20 Mol-% der Säuregruppen enthaltenden Monomeren (a) mit tertiären Alkanolaminen neutralisiert und/oder daß die freien Säuregruppen des schaumförmigen Hydrogels nach dem Polymerisieren zu mindestens 20 Mol-% mit mindestens einem Alkanolamin neutralisiert worden sind.

Gegenstand der Erfindung ist außerdem ein Verfahren zur Herstellung von wasserabsorbierenden, schaumförmigen, vernetzten Polymerisaten, wobei man eine polymerisierbare Mischung aus

(a) Säuregruppen enthaltenden monoethylenisch ungesättigten Monomeren, die gegebenenfalls neutralisiert sind,

(b) gegebenenfalls anderen monoethylenisch ungesättigten Monomeren,

(c) Vernetzer,

(d) Initiatoren,

(e) 0,1 bis 20 Gew.-% mindestens einem Tensid,

(f) gegebenenfalls mindestens einem Lösevermittler und

(g) gegebenenfalls Verdickern, Schaumstabilisatoren, Polymerisationsreglern, Füllstoffen und/oder Zellkeimbildnern

in einer ersten Verfahrensstufe durch Dispergieren von feinen Blasen eines gegenüber Radikalen inerten Gases schäumt und den so erhaltenen Schaum in einer zweiten Verfahrensstufe unter Bildung eines schaumförmigen Hydrogels polymerisiert und gegebenenfalls den Wassergehalt des schaumförmigen Hydrogels auf 1 bis 60 Gew.-% einstellt, das dadurch gekennzeichnet ist, daß man mindestens 20 Mol-% der Säuregruppen enthaltenden Monomeren (a) mit tertiären Alkanolaminen neutralisiert und/oder daß man die freien Säuregruppen des schaumförmigen Hydrogels nach dem Polymerisieren zu mindestens 20 Mol-% mit mindestens einem Alkanolamin neutralisiert.

Durch diese Vorgehensweise werden nicht nur im oben genannten Klimabereich lagerstabile, flexible Schäume erhalten, sondern ihre Aufnahmegeschwindigkeiten für Körperflüssigkeiten sind deutlich verbessert. Außerdem wird überraschenderweise durch diese Maßnahme zusätzlich die Aufnahmekapazität vergrößert und auch das Problem des feuchten Griffs beseitigt. Die extreme Klebrigkeit kann durch eine zusätzliche Puderung mit feinteiligen, hydrophilen Pulvern gelöst werden. Weiterhin bietet der vorliegende Schaum die Möglichkeit, die Alkanolamine erst nachträglich, d.h. nach der Polymerisation, aufzubringen z.B. durch Aufsprühen. Für die oben geschilderten Filme ist dies nicht praktikabel, da die Zeiten, die für die Aufnahme in den Film benötigt werden, für eine praktische Anwendung nicht brauchbar sind und eine homogene Neutralisationsreaktion nicht zu erreichen ist. Die Möglichkeit der "Nachneutralisation" erweitert die Palette der anwendbaren Alkanolamine auf sekundäre und primäre Typen. Ein Zusatz dieser beiden Alkanolamintypen zu Monomermischung, wie in JP-A-08073507 beschrieben, ist nicht möglich, weil sie mit Acrylaten unter Michael-Addition abreagieren.

Erfindungsgemäß wird eine polymerisierbare wäßrige Mischung zu einem Schaum verarbeitet, der verarbeitungsstabil ist und beliebig geformt werden kann. Die polymerisierbare wäßrige Mischung enthält als Komponenten (a) Säuregruppen enthaltende monoethylenisch ungesättigte Monomere, die gegebenenfalls neutralisiert sind. Solche Monomere sind beispielsweise monoethylenisch ungesättigte $C_3$- bis $C_{25}$-Carbonsäuren oder Anhydride, beispielsweise Acrylsäure, Methacrylsäure, Ethacrylsäure, $\alpha$-Chloracrylsäure, Crotonsäure, Maleinsäure, Maleinsäureanhydrid, Itaconsäure, Citraconsäure, Mesaconsäure, Glutaconsäure, Aconitsäure und Fumarsäure.

Als Monomere der Gruppe (a) kommen außerdem monoethylenisch ungesättigte Sulfonsäuren in Betracht, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Vinylphosphorsäure, Allylphosphonsäure und 2-Acrylamido-2-methylpropansulfonsäure. Die Monomeren können allein oder in Mischung untereinander bei der Herstellung der superabsorbierenden Schäume eingesetzt werden. Bevorzugt eingesetzte Monomere der Gruppe (a) sind Acrylsäure, Methacrylsäure, Vinylsulfonsäure, Acrylamidopropansulfonsäure oder Mischungen dieser Säuren, z.B. Mischungen aus Acrylsäure und Methacrylsäure, Mischungen aus Acrylsäure und Acrylamidopropansulfonsäure oder Mischungen aus Acrylsäure und Vinylsulfonsäure.

Die Monomeren sind gegebenenfalls neutralisiert. Zur Neutralisation verwendet man beispielsweise Alkalimetallbasen oder Ammoniak bzw. Amine. Zur Neutralisation setzt man vorzugsweise Natronlauge oder Kalilauge ein. Die Neutralisation kann jedoch auch mit Hilfe von Natriumcarbonat, Natriumhydrogencarbonat, Kaliumcarbonat oder Kaliumhydrogencarbonat oder anderen Carbonaten oder Hydrogencarbonate oder Ammoniak vorgenommen werden. Die Säuregruppen der Monomeren werden vorzugsweise zu 15 bis 40 Mol-% mit mindestens einer der oben angegebenen Basen neutralisiert.

Bei einer Ausführungsform des erfindungsgemäßen Verfahrens neutralisiert man die Monomeren (a) zu mindestens 20 Mol-% mit tertiären Alkanolaminen. Besonders bevorzugt ist eine Ausführungsform, bei der man mindestens 40 Mol-% der Säuregruppen enthaltenden Monomeren (a) mit tertiären Alkanolaminen neutralisiert. Bei dieser Ausführungsform des erfindungsgemäßen Verfahrens kann man die Monomeren (a) gegebenenfalls zusätzlich mit den oben beschriebenen Basen, insbesondere NaOH oder Ammoniak, bis zu beispielsweise 100 % neutralisieren. Der Neutralisationsgrad der Säuregruppen enthaltenden Monomeren (a) mit tertiären Alkanolaminen beträgt bei dieser Verfahrensvariante 20 bis 95, vorzugsweise 30 bis 70 Mol-%. Bevorzugt verwendete tertiäre Amine sind Triethanolamin, Methyldiethanolamin, Dimethylaminodiglykol, Dimethylethanolamin und N,N,N',N'-Tetra-(hydroxyethyl)ethylendiamin. Eine weitere Ausführungsform des erfindungsgemäßen Verfahrens wird weiter unten beschrieben.

Die polymerisierbare wäßrige Mischung kann gegebenenfalls Monomere der Gruppe (b) enthalten. Hierunter sollen andere monoethylenisch ungesättigte Monomere verstanden werden, die mit den Monomeren (a) und (c) copolymerisierbar sind. Hierzu gehören beispielsweise die Amide und Nitrile von monoethylenisch ungesättigten Carbonsäuren, z.B. Acrylamid, Methacrylamid und N-Vinylformamid, Acrylnitril und Methacrylnitril, Dialkyldiallylammoniumhalogenide wie Dimethyldiallylammoniumchlorid, Diethyldiallylammoniumchlorid, Allylpiperidiniumbromid, N-Vinylimidazole wie z.B. N-Vinylimidazol, 1-Vinyl-2-methylimidazol und N-Vinylimidazoline wie N-Vinylimidazolin, 1-Vinyl-2-methylimidazolin, 1-Vinyl-2-ethylimidazolin oder 1-Vinyl-2-propylimidazolin, die in Form der freien Basen, in quaternisierter Form oder als Salz bei der Polymerisation eingesetzt werden können. Außerdem eignen sich Dialkylaminoalkylacrylate und Dialkylaminoalkylmethacrylate, Dimethylaminoethylacrylat, Dimethylaminoethylmethacrylat, Diethylaminoethylacrylat und Diethylaminoethylmethacrylat. Die basischen Estern werden vorzugsweise in quaternisierter Form oder als Salz eingesetzt. Weitere geeignete Verbindungen der Gruppe (b) sind beispielsweise Vinylester von gesättigten $C_1$- bis $C_4$-Carbonsäuren wie Vinylformiat, Vinylacetat oder Vinylpropionat, Alkylvinylether mit mindestens 2 C-Atomen in der Alkylgruppe, wie z.B. Ethylvinylether oder Butylvinylether, Ester von monoethylenisch ungesättigten $C_3$- bis $C_6$-Carbonsäuren, z.B. Ester aus einwertigen $C_1$- bis $C_{18}$-Alkoholen und Acrylsäure, Methacrylsäure oder Maleinsäure, Halbester von Maleinsäure, z.B. Maleinsäuremonomethylester und Hydroxyalkylester der genannten monoethylenisch ungesättigten Carbonsäuren, z.B. 2-Hydroxyethylacrylat, Hydroxypropylacrylat, Hydroxybutylacrylat, Hydroxyethylmethacrylat, Hydroxypropylmethacrylat und Hydroxybutylmethacrylat, N-Vinyllactame wie N-Vinylpyrrolidon oder N-Vinylcaprolactam, Acrylsäureund Methacrylsäureester von alkoxylierten einwertigen, gesättigten Alkoholen, z.B. von Alkoholen mit 10 bis 25 C-Atomen, die mit 2 bis 200 Mol Ethylenoxid und/oder Propylenoxid pro Mol Alkohol umgesetzt worden sind, sowie Monoacrylsäureester und Monomethacrylsäureester von Polyethylenglykol oder Polypropylenglykol, wobei die Molmassen ($M_N$) der Polyalkylenglykole beispielsweise bis zu 2000 betragen können. Weiterhin geeignete Monomere der Gruppe (b) sind alkylsubstituierte Styrole wie Ethylstyrol oder tert. Butylstyrol. Die Monomeren der Gruppe (b) können auch in Mischung bei der Copolymerisation mit den anderen Monomeren eingesetzt werden, z.B. Mischungen aus Vinylacetat und 2-Hydroxyethylacrylat in beliebigem Verhältnis.

Die Monomeren der Gruppe (c) haben mindestens 2 ethylenisch ungesättigte Doppelbindungen. Beispiele für solche Monomere, die bei Polymerisationsreaktionen üblicherweise als Vernetzer eingesetzt werden, sind N,N'-Methylen-bisacrylamid, Polyethylenglykoldiacrylate und Polyethylenglykoldimethacrylate, die sich jeweils von Polyethylenglykolen eines Molekulargewichts von 106 bis 8500, vorzugsweise 400 bis 2000, ableiten, Trimethylolpropantriacrylat, Trimethylolpropantrimethacrylat, Ethylenglykoldiacrylat, Propylenglykoldiacrylat, Butandioldiacrylat, Hexandioldiacrylat, Hexandioldimethacrylat, Diacrylate und Dimethacrylate von Blockcopolymerisaten aus Ethylenoxid und Propylenoxid, zweifach bzw. dreifach mit Acrylsäure oder Methacrylsäure veresterte mehrwertige Alkohole, wie Glycerin oder Pentaerythrit, Triallylamin, Tetralylethylendiamin, Divinylbenzol, Diallylphthalat, Polyethylenglykoldivinylether von Polyethylenglykolen eines Molekulargewichts von 126 bis 4000, Trimethylolpropandialylether, Butandioldivinylether, Pentaerythrittriallylether und/oder Divinylethylenharnstoff. Vorzugsweise setzt man wasserlösliche Vernetzer ein, z.B. N,N'-Methylen-bisacrylamid, Polyethylenglykoldiacrylate und Polyethylenglykoldimethacrylate, die sich von Additionsprodukten von 2 bis 400 Mol Ethylenoxid an 1 Mol eines Diols oder Polyols ableiten, Vinylether von Additionsprodukten von 2 bis 400 Mol Ethylenoxid an 1 Mol eines Diols oder Polyols, Ethylenglykoldiacrylat, Ethylenglykoldimethacrylat oder Triacrylate und Trimethacrylate von Additionsprodukten von 6 bis 20 Mol Ethylenoxid an ein Mol Glycerin, Pentaerythrittriallylether und/oder Divinylharnstoff.

Als Vernetzer kommen außerdem Verbindungen in Betracht, die mindestens eine polymerisierbare ethylenisch ungesättigte Gruppe und mindestens eine weitere funktionelle Gruppe enthalten. Die funktionelle Gruppe dieser Vernetzer muß in der Lage sein, mit den funktionellen Gruppen, im wesentlichen den Carboxylgruppen oder Sulfonsäuregruppen der Monomeren (a) zu reagieren. Geeignete funktionelle Gruppen sind beispielsweise Hydroxyl-, Amino-, Epoxi- und Aziridinogruppen.

5 Als Vernetzer kommen außerdem solche Verbindungen in Betracht, die mindestens zwei funktionelle Gruppen tragen, die mit Carboxyl- und Sulfonsäuregruppen der eingesetzten Monomeren der Gruppe (a) reagieren können. Die geeigneten funktionellen Gruppen wurden bereits oben genannt, d.h. Hydroxyl-, Amino-, Epoxi-, Isocyanat-, Ester-, Amide- und Aziridinogruppen. Beispiele für solche Vernetzer sind Ethylenglykol, Diethylenglykol, Triethylenglykol, Tetraethylenglykol, Polyethylenglykol, Glycerin, Polyglycerin, Propylenglykol, Polypropylenglykol, Blockcopolymerisate aus Ethylenoxid und Propylenoxid, Sorbitanfettsäureester, ethoxylierte Sorbitanfettsäureester, Trimethylolpropan, Pentaerythrit, Polyvinylalkohol, Sorbit, Polyglycidylether wie Ethylenglykoldiglycidylether, Polyethylenglykoldiglycidylether, Glycerindiglycidylecher, Glycerinpolyglycidylether, Diglycerinpolyglycidylether, Polyglycerinpolyglycidylether, Sorbitpolyglycidylether, Pentaerythritpolyglycidylether, Propylenglykoldiglycidylether und Polypropylenglykoidiglycidylether, Polyaziridinverbindungen wie 2,2-Bishydroxymethylbutanol-tris[3-(1-aziridinyl)propionat], 1,6-Hexamethylendiethylenharnstoff, Diphenylmethan-bis-4,4' -N,N'diethylenharnstoff, Halogenepoxyverbindungen wie Epichlorhydrin und $\alpha$-Methylfluorhydrin, Polyisocyanate wie 2,4-Toluylendiisocyanat und Hexamethylendiisocyanat, Alkylencarbonate wie 1,3-Dioxolan-2-on und 4- Methyl-1,3-dioxolan-2-on, polyquaternäre Amine wie Kondensationsprodukte von Dimethylamin mit Epichlorhydrin, Homo- und Copolymere von Diallyldimethylammoniumchlorid sowie Homo- und Copolymerisate von Dimethylaminoethyl(meth)acrylat, die gegebenenfalls mit beispielsweise Methylchlorid quaterniert sind.

Weitere geeignete Vernetzer sind polyvalente Metallionen, die in der Lage sind, ionische Vernetzungen auszubilden. Beispiele für solche Vernetzer sind Magnesium-, Calcium-, Barium- und Aluminiumionen. Ein bevorzugter Vernetzer dieser Art ist Natriumaluminat. Diese Vernetzer werden beispielsweise als Hydroxide, Carbonate oder Hydrogencarbonate der wäßrigen polymerisierbaren Lösung zugesetzt.

Weitere geeignete Vernetzer sind multifunktionelle Basen, die ebenfalls in der Lage sind, ionische Vernetzungen auszubilden, beispielsweise Polyamine oder deren quaternierte Salze. Beispiele für Polyamine sind Ethylendiamin, Diethylentriamin, Triethylentetramin, Tetraethylenpentamin, Pentaethylenhexamin und Polyethylenimine sowie Polyvinylamine mit Molmassen von jeweils bis zu 4000000.

In einer bevorzugten Ausführungsform der Erfindung werden zwei unterschiedliche Vernetzer eingesetzt, von denen der eine wasserlöslich und der andere wasserunlöslich ist. Der hydrophile Vernetzer, der in der wäßrigen Phase der Reaktionsmischung löslich ist, bewirkt in konventioneller weise eine relativ gleichmäßige Vernetzung des entstehenden Polymeren, wie es bei der Herstellung eines Superabsorbers üblich ist. Der hydrophobe Vernetzer, der in der polymerisierbaren wäßrigen Mischung unlöslich bzw. darin nur begrenzt löslich ist, reichert sich in der Tensidgrenzschicht zwischen der Gasphase und der polymerisierbaren wäßrigen Phase an. Dadurch wird bei der nachfolgenden Polymerisation die Oberfläche des Schaums stärker vernetzt als der innere Teil des Superabsorberhydrogels. Man erhält dadurch einen Kern-Schale-Aufbau des Schaums unmittelbar bei der Herstellung des Superabsorberschaums. Eine solche starke oberflächliche Vernetzung eines Superabsorberschaums ist bei den bekannten Herstellverfahren des Standes der Technik nur dadurch möglich, daß man einen bereits gebildeten schaumförmigen Superabsorber nachträglich oberflächlich vernetzt. Für diese Nachvernetzung ist bei konventioneller Arbeitsweise ein eigener Prozeßschritt notwendig, der bei dem Verfahren der vorliegenden Erfindung entfallen kann.

Produkte mit einem Kern-Schale-Aufbau zeigen gegenüber homogen vernetzten Proben hinsichtlich der Aufnahmegeschwindigkeit, Verteilungswirkung und Gelstabilität deutlich verbesserte Eigenschaften. Mit Ausnahme polyvalenter Metallionen sind alle oben beschriebenen wasserunlöslichen Vernetzer, die den unterschiedlichen Gruppen zugeordnet werden können, geeignet, um Schäume mit einem Kern-Schale-Aufbau herzustellen, d.h. Schäume, bei denen die gesamte Oberfläche stärker vernetzt ist als die darunter liegende Schicht, die oben als Kernschicht bezeichnet wurde. Besonders bevorzugte hydrophobe Vernetzer sind Diacrylate oder Dimethacrylate oder Divinylether von Alkandiolen mit 2 bis 25 C-Atomen (verzweigt, linear, mit beliebiger Anordnung der OH-Gruppen) wie z.B. 1,3-Propandiol, 1,4-Butandiol, 1,6-Hexandiol, Neopentylglycol, 1,9-Nonandiol oder 1,2-Dodecandiol, Di-, Tri- oder Polypropylenglycoldiacrlyate oder -dimethacrylate, Allylacrylat, Allylmethacrylat, Divinylbenzol, Glycidylacrylat oder Glycidylmethacrylat, Allylglycidylether und Bisglycidylether der oben aufgeführten Alkandiole.

Geeignete hydrophile Vernetzer sind beispielsweise N,N'-Methylenbisacrylamid, Polyethylenglycoldiacrylate oder -dimethacrylate mit einem Molekulargewicht $M_N$ von 200 bis 4000, Dinvinylharnstoff, Triallylamin, Diacrylate oder Dimethacrylate von Additionsprodukten von 2 bis 400 Mol Ethylenoxid an 1 Mol eines Diols oder Polyols oder das Triacrylat eines Additionsprodukts von 20 Mol Ethylenoxid an 1 Mol Glycerin und Vinylether von Additionsprodukten von 2 bis 400 Mol Ethylenoxid an 1 Mol eines Diols oder Polyols.

Die Monomeren der Gruppe (a) sind in der polymerisierbaren wäßrigen Mischung beispielsweise in Mengen von 10 bis 90 und vorzugsweise 20 bis 85 Gew.-% enthalten. Die Monomeren der Gruppe (b) werden nur gegebenenfalls zur Modifizierung der Superabsorberschäume eingesetzt und können in Mengen bis zu 50, vorzugsweise in Mengen bis zu 20 Gew.-% in der polymerisierbaren wäßrigen Mischung enthalten sein. Die Vernetzer (c) sind in der Reaktionsmischung beispielsweise von 0,001 bis 8 und vorzugsweise von 0,01 und 5 Gew.-% vorhanden.

Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wäßrige Mischung ist möglich. Die Polymerisation kann allerdings auch in Abwesenheit von Initiatoren der obengenannten Art durch Einwirkung energiereicher Strahlung in Gegenwart von Photoinitiatoren ausgelöst werden.

Als Polymerisationsinitiatoren können sämtliche unter den Polymerisationsbedingungen in Radikale zerfallende Verbindungen eingesetzt werden, z.B. Peroxide, Hydroperoxide. Wasserstoffperoxid, Persulfate, Azoverbindungen und die sogenannten Redoxkatalysatoren. Bevorzugt ist der Einsatz von wasserlöslichen Initiatoren. In manchen Fällen ist es vorteilhaft, Mischungen verschiedener Polymerisationsinitiatoren zu verwenden, z.B. Mischungen aus Wasserstoffperoxid und Natrium- oder Kaliumperoxidisulfat. Mischungen aus Wasserstoffperoxid und Natriumperoxidisulfat können in jedem beliebigen Verhältnis verwendet werden. Geeignete organische Peroxide sind beispielsweise Acetylacetonperoxid, Methylethylketonperoxid, tert.-Butylhydroperoxid, Cumolhydroperoxid, tert.-Amylperpivalat, tert.-Butylperpivalat, tert.-Butylperneohexanoat, tert.-Butylperisobutyrat, tert.-Butylper-2-ethylhexanoat, tert.-Butylperisononanoat, tert.-Butylpermaleat, tert.-Butylperbenzoat, Di-(2-ethylhexyl)-peroxidicarbonat, Dicyclohexylperoxydicarbonat, Di-(4-tert.-butylcyclohexyl)peroxidicarbonat, Dimyristil-peroxidicarbonat, Diacetylperoxydicarbonat, Allylperester, Cumylperoxyneodecanoat, tert.-Butylper-3,5,5-tri-methylhexanoat, Acetylcyclohexylsulfonylperoxid, Dilaurylperoxid, Dibenzoylperoxid und tert.-Amylperneodekanoat. Besonders geeignete Polymerisationsinitiatoren sind wasserlösliche Azostarter, z.B. 2,2'-Azobis-(2-amidinopropan)dihydrochlorid, 2,2'-Azobis-(N,N'-dimethylen)isobutyramidin-dihydrochlorid, 2-(Carbamoylazo)isobutyronitril, 2,2'-Azobis[2-(2'-imidazolin-2-yl)propan]dihydrochlorid und 4,4'-Azobis-(4-cyanovaleriansäure). Die genannten Polymerisationsinitiatoren werden in üblichen Mengen eingesetzt, z.B. in Mengen von 0,01 bis 5, vorzugsweise 0,1 bis 2,0 Gew.-%, bezogen auf die zu polymerisierenden Monomeren.

Als Initiatoren kommen außerdem Redoxkatalysatoren in Betracht. Die Redoxkatalysatoren enthalten als oxidierende Komponente mindestens eine der oben angegebenen Perverbindungen und als reduzierende Komponente beispielsweise Ascorbinsäure, Glukose, Sorbose, Ammonium- oder Alkalimetall-hydrogensulfit, -sulfit, -thiosulfat, -hyposulfit, -pyrosulfit oder -sulfid, Metallsalze, wie Eisen-II-ionen oder Silberionen oder Natriumhydroxymethylsulfoxylat. Vorzugsweise verwendet man als reduzierende Komponente des Redoxkatalysators Ascorbinsäure oder Natriumsulfit. Bezogen auf die bei der Polymerisation eingesetzte Menge an Monomeren verwendet man beispielsweise $3 \cdot 10^{-6}$ bis 1 Mol-% der reduzierenden Komponente des Redoxkatalysatorsystems und 0,001 bis 5,0 Mol-% der oxidierenden Komponente des Redoxkatalysators.

Wenn man die Polymerisation durch Einwirkung energiereicher Strahlung auslöst, verwendet man üblicherweise als Initiator sogenannte Photoinitiatoren. Hierbei kann es sich beispielsweise um sogenannte α-Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanton-Derivate, Cumarin-Derivate, Benzoinether und deren Derivate, Azoverbindungen wie die oben genannten Radikalbildner, substituierte Hexaarylbisimidazole oder Acylphosphinoxide. Beispiele für Azide sind: 2-(N,N-Dimethylamino)-ethyl-4-azidocinnamat, 2-(N,N-Dimethylamino)-ethyl-4-azidonaphthylketon, 2-(N,N-Dimethylamino)-ethyl-4-azidobenzoat, 5-Azido-1-naphthyl-2'-(N,N-dimethylamino)ethylsulfon, N-(4-Sulfonylazidophenyl)maleinimid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclohexanon und 2,6-Bis(p-azidobenzyliden)-4-methylcyclohexanon. Die Photoinitiatoren werden, falls sie eingesetzt werden, üblicherweise in Mengen von 0,01 bis 5 Gew.-%, bezogen auf die zu polymerisierenden Monomeren angewendet.

Die polymerisierbaren wäßrigen Mischungen enthalten als Komponente (e) 0,1 bis 20 Gew.-% mindestens eines Tensids. Die Tenside sind für die Herstellung und die Stabilisierung des Schaums von entscheidender Bedeutung. Man kann anionische, kationische oder nichtionische Tenside oder Tensidmischungen verwenden, die miteinander verträglich sind. Man kann niedermolekulare oder auch polymere Tenside einsetzen, wobei sich Kombinationen unterschiedlicher oder auch gleichartiger Typen von Tensiden als vorteilhaft herausgestellt haben. Nichtionische Tenside sind beispielsweise Additionsprodukte von Alkylenoxiden, insbesondere Ethylenoxid, Propylenoxid und/oder Butylenoxid an Alkohole, Amine, Phenole, Naphthole oder Carbonsäuren. Vorteilhaft setzt man als Tenside Additionsprodukte von Ethylenoxid und/oder Propylenoxid an mindestens 10 C-Atome enthaltende Alkohole ein, wobei die Additionsprodukte pro Mol Alkohol 3 bis 200 Mol Ethylenoxid und/oder Propylenoxid angelagert enthalten. Die Additionsprodukte enthalten die Alkylenoxid-Einheiten in Form von Blöcken oder in statistischer Verteilung. Beispiele für nichtionische Tenside sind die Additionsprodukte von 7 Mol Ethylenoxid an 1 Mol Talgfettalkohol.

Umsetzungsprodukte von 9 Mol Ethylenoxid mit 1 Mol Talgfettalkohol und Additionsprodukte von 80 Mol Ethylenoxid an 1 Mol Talgfettalkohol. Weitere handelsübliche nichtionische Tenside bestehen aus Umsetzungsprodukten von Oxoalkoholen oder Ziegler-Alkoholen mit 5 bis 12 Mol Ethylenoxid pro Mol Alkohol, insbesondere mit 7 Mol Ethylenoxid. Weitere handelsübliche nichtionische Tenside werden durch Ethoxylierung von Rizinusöl erhalten. Pro Mol Rizinusöl werden beispielsweise 12 bis 80 Mol Ethylenoxid angelagert. Weitere handelsübliche Produkte sind beispielsweise die Umsetzungsprodukte von 18 Mol Ethylenoxid mit 1 Mol Talgfettalkohol, die Additionsprodukte von 10 Mol Ethylenoxid an 1 Mol eines $C_{13}/C_{15}$-Oxoalkohols, oder die Umsetzungsprodukte von 7 bis 8 Mol Ethylenoxid an 1 Mol eines $C_{13}/C_{15}$-Oxoalkohols. Weitere geeignete nichtionische Tenside sind Phenolalkoxylate wie beispielsweise p-tert.-Butylphenol, das mit 9 Mol Ethylenoxid umgesetzt ist, oder Methylether von Umsetzungsprodukten aus 1 Mol eines $C_{12}$- bis $C_{18}$-Alkohols und 7,5 Mol Ethylenoxid.

Die oben beschriebenen nichtionischen Tenside können beispielsweise durch Veresterung mit Schwefelsäure in die entsprechenden Schwefelsäurehalbester überführt werden. Die Schwefelsäurehalbester werden in Form der Alkalimetall- oder Ammoniumsalze als anionische Tenside eingesetzt. Als anionische Tenside eignen sich beispielsweise Alkalimetall- oder Ammoniumsalze von Schwefelsäurehalbestern von Additionsprodukten von Ethylenoxid und/oder Propylenoxid an Fettalkohole, Alkalimetall- oder Ammoniumsalze von Alkylbenzolsulfonsäure oder von Alkylphenolethersulfaten. Produkte der genannten Art sind im Handel erhältlich. Beispielsweise sind das Natriumsalz eines Schwefelsäurehalbesters eines mit 106 Mol Ethylenoxid umgesetzten $C_{13}/C_{15}$-Oxoalkohols, das Triethanolaminsalz von Dodecylbenzolsulfonsäure, das Natriumsalz von Alkylphenolethersulfaten und das Natriumsalz des Schwefelsäurehalbesters eines Umsetzungsprodukts von 106 Mol Ethylenoxid mit 1 Mol Talgfettalkohol handelsübliche anionische Tenside. Weitere geeignete anionische Tenside sind Schwefelsäurehalbester von $C_{13}/C_{15}$-Oxoalkoholen, Paraffinsulfonsäuren wie $C_{15}$-Alkylsulfonat, alkylsubstituierte Benzolsulfonsäuren und alkylsubstituierte Naphthalinsulfonsäuren wie Dodecylbenzolsulfonsäure und Di-n-butylnaphthalinsulfonsäure sowie Fetcalkoholphosphate wie $C_{15}/C_{18}$-Fettalkoholphosphat. Die polymerisierbare wäßrige Mischung kann Kombinationen aus einem nichtionischen Tensid und einem anionischen Tensid oder Kombinationen aus nichtionischen Tensiden oder Kombinationen aus anionischen Tensiden enthalten. Auch kationische Tenside sind geeignet. Beispiele hierfür sind die mit Dimethylsulfat quaternierten Umsetzungsprodukte von 6,5 Mol Ethylenoxid mit 1 Mol Oleylamin, Distearyldimethylammoniumchlorid, Lauryltrimethylammoniumchlorid, Cetylpyridiniumbromid und mit Dimethylsulfat quaternierter Stearinsäuretriethanolaminester, der bevorzugt als kationisches Tensid eingesetzt wird.

Der Tensidgehalt der polymerisierbaren wäßrigen Mischung beträgt 0,1 bis 20, vorzugsweise 0,5 bis 10 Gew.-%. In den meisten Fällen weisen die polymerisierbaren wäßrigen Mischungen einen Tensidgehalt von 1,5 bis 8 Gew.-% auf.

Die polymerisierbaren wäßrigen Mischungen können als Komponente (f) gegebenenfalls mindestens einen Lösevermittler enthalten. Hierunter sollen mit Wasser mischbare organische Lösemittel verstanden werden, z.B. Alkohole, Glykole, Polyethylenglykole bzw. davon abgeleitete Monoether, wobei die Monoether keine Doppelbindungen im Molekül enthalten. Geeignete Ether sind Methylglykol, Butylglykol, Butyldiglykol, Methyldiglykol, Butyltriglykol, 3-Ethoxy-1-propanol und Glycerinmonomethylether.

Die polymerisierbaren wäßrigen Mischungen enthalten 0 bis 50 Gew.-% mindestens eines Lösevermittlers. Falls Lösevermittler eingesetzt werden, beträgt ihr Gehalt in der polymerisierbaren wäßrigen Mischung vorzugsweise bis 25 Gew.-%.

Die polymerisierbare wäßrige Mischung kann gegebenenfalls Verdicker, Schaumstabilisatoren, Polymerisationsregler, Füllstoffe und Zellkeimbildner enthalten. Verdicker werden beispielsweise zur Optimierung der Schaumstruktur und zur Verbesserung der Schaumstabilität eingesetzt. Man erreicht damit, daß der Schaum während der Polymerisation nur geringfügig schrumpft. Als Verdickungsmittel kommen alle hierfür bekannten natürlichen und synthetischen Polymeren in Betracht, die die Viskosität eines wäßrigen Systems stark erhöhen. Hierbei kann es sich um wasserquellbare oder wasserlösliche synthetische und natürliche Polymere handeln. Als Verdicker sind auch pulverförmige Superabsorber geeignet. Eine ausführliche Übersicht über Verdicker findet man beispielsweise in den Veröffentlichungen von R.Y. Lochhead und W.R. Fron, Cosmetics & Toileteris, 108, 95-135 (Mai 1993) und M.T. Clarke, "Rheological Additives" in D. Laba (ed.) "Rheological Properties of Cosmetics and Toiletris", Cosmetic Science and Technology Series, Vol. 13, Marcel Dekker Inc., New York 1993.

Als Verdicker in Betracht kommende wasserquellbare oder wasserlösliche synthetische Polymere sind beispielsweise hochmolekulare Polymerisate der oben unter (a) beschriebenen Säuregruppen enthaltenden monoethylenisch ungesättigten Monomeren. Solche Verdicker sind beispielsweise hochmolekulare Homopolymerisate von Acrylsäure und/oder Methacrylsäure oder geringfügig vernetzte Copolymerisate aus Acrylsäure und/oder Methacrylsäure und einer Verbindung, die mindestens 2 ethylenisch ungesättigte Doppelbindungen enthält, z.B. Butandioldiacrylat. Außerdem eignen sich hochmolekulare Polymerisate von Acrylamid und Methacrylamid oder Copolymerisate aus Acrylsäure und Acrylamid mit Molmassen von mehr als 1 Million. Solche Copolymerisate sind als Verdickungsmittel bekannt. Auch hochmolekulare Polyethylenglykole oder Copolymerisate aus Ethylenglykol und Propylenglykol sowie hochmolekulare Polysaccharide wie Stärke, Guarkernmehl, Johannisbrotkernmehl oder Derivate von Naturstoffen wie Carboxymethylcellulose Hydroxyethylcellulose, Hydroxymethylcellulose, Hydroxypropylcellulose und Cellulose Mischether sind bekannte Verdicker. Eine weitere Gruppe von Verdickern sind wasserunlösliche Produkte, wie feinteiliges Siliciumdioxid, pyrogene Kieselsäuren, Fällungskieselsäuren in hydrophilen oder hydrophoben Modifikationen, Zeolithe, Titandioxid, Cellulosepulver, oder andere von Superabsorbern verschiedene feinteilige Pulver von vernetzten Polymerisaten. Die polymerisierbaren wäßrigen Mischungen können die Verdicker in Mengen bis zu 30 Gew.-% enthalten. Falls solche Verdickungsmittel überhaupt eingesetzt werden, sind sie in Mengen von 0,1, vorzugsweise 0,5 bis 20 Gew.-% in der polymerisierbaren wäßrigen Mischung enthalten.

Um die Schaumstruktur zu optimieren, kann man gegebenenfalls Kohlenwasserstoffe mit mindestens 5 C-Atomen im Molekül zu der wäßrigen Reaktionsmischung zusetzen. Geeignete Kohlenwasserstoffe sind beispielsweise Pentan, Hexan, Cyclohexan, Heptan, Octan, Isooctan, Decan und Dodecan. Die in Betracht kommenden aliphatischen Kohlenwasserstoffe können geradkettig, verzweigt oder zyklisch sein und haben eine Siedetemperatur, die oberhalb der Temperatur der wäßrigen Mischung während des Schäumens liegt. Die aliphatischen Kohlenwasserstoffe erhöhen die Standzeit der noch nicht polymerisierten geschäumten wäßrigen Reaktionsmischung. Dadurch wird das Handling der noch nicht polymerisierten Schäume erleichtert und die Prozeßsicherheit erhöht. Die Kohlenwasserstoffe werden in Mengen von 0 bis 10 Gew.-%, bezogen auf die polymerisierbare wäßrige Mischung eingesetzt. Im Fall ihres Einsatzes betragen die bevorzugt in der wäßrigen Mischung vorliegenden Mengen 0,1 bis 5 Gew.-%.

Um die Eigenschaften der Superabsorber zu variieren, beispielsweise die Aufnahmegeschwindigkeit und die Aufnahmekapazität von Wasser, kann es von Vorteil sein, der wäßrigen Reaktionsmischung einen Polymerisationsregler oder eine Mischung mehrerer Polymerisationsregler zuzusetzen. Geeignete Polymerisationsregler sind beispielsweise Ameisensäure, Thioverbindungen wie 2-Mercaptoethanol, Mercaptopropanol, Mercaptobutanol, Dodecylmercaptan, Thioglykolsäure oder Amine wie Triethylamin, Morpholin oder Piperidin. Die Mengen an Polymerisationsregler können bis zu 10 Gew.-%, bezogen auf die eingesetzten Monomeren, betragen. Falls Polymerisationsregler eingesetzt werden, verwendet man vorzugsweise 0,1 bis 5 Gew.-%, bezogen auf die Monomeren.

Die unter (g) angegebenen fakultativ zu verwendenden Bestandteile können einzeln, oder in Mischung bei der Herstellung der erfindungsgemäßen Polymerisate eingesetzt werden. Man kann jedoch auch in Abwesenheit von Verdickern, Schaumstabilisatoren, Füllstoffe, Zellkeimbildner und Polymerisationsreglern arbeiten.

Bei der erfindungsgemäßen Herstellung von wasserabsorbierenden, schaumförmigen, vernetzten Polymerisaten wird in einer ersten Verfahrensstufe die oben beschriebene polymerisierbare wäßrige Mischung geschäumt. Zu diesem Zweck wird in der wäßrigen Monomerphase ein gegenüber Radikalen inertes Gas in Form von feinen Blasen in der Art dispergiert, daß sich ein Schaum bildet. Das Eintragen von Gasblasen in die Monomermischung gelingt beispielsweise mit Hilfe von Schlag-, Schüttel-, Rühr- oder Peitschvorrichtungen. Ferner ist es möglich solche Schäume dadurch herzustellen, daß Gase aus einer flüssigkeitsbedeckten Öffnung ausströmen oder durch das Ausnutzen von Turbulenzerscheinungen in Strömungen. Schließlich kann auch die Ausbildung von Lamellen an Drähten oder Sieben für diesen Zweck genutzt werden. Diese unterschiedlichen Methoden können auch gegebenenfalls miteinander kombiniert werden. Als gegenüber Radikalen inerte Gase eignen sich beispielsweise Stickstoff, Kohlendioxid, Helium, Neon und Argon. Vorzugsweise verwendet man Stickstoff.

Die Herstellung des Schaums erfolgt erfindungsgemäß getrennt von der Polymerisation. Die polymerisierbare wäßrige Mischung kann beispielsweise in technischen Apparaturen geschäumt werden, die für die Herstellung von Harnstoff-Formaldehyd-Schäumen bekannt sind, vgl. Frisch und Saunders, Polymeric Foams Part II, S. 679 ff (1973). Das Schäumen der polymerisierbaren wäßrigen Mischung kann im Labor im einfachsten Fall in einer konventionellen Küchenmaschine erfolgen, die mit Schneebesen bestückt ist. Die Schlagschaumerzeugung wird vorzugsweise in einer Inertgasatmosphäre durchgeführt. Als Inertgase sind beispielsweise Stickstoff, Edelgase oder Kohlendioxid verwendbar. Zur Herstellung des Schaums werden alle Komponenten der Reaktionsmischung vereinigt. Zweckmäßigerweise geht man dabei so vor, daß zunächst alle wasserlöslichen Komponenten in Wasser gelöst werden und daß man erst danach die wasserunlöslichen Stoffe zusetzt. Je nach verwendetem Verfahren der Schlagschaumerzeugung und in Abhängigkeit von dem in der polymerisierbaren wäßrigen Mischung enthaltenen Initiator kann es auch von Vorteil sein, den Initiator erst am Ende des Aufschlagprozesses der Mischung zuzusetzen. Die Konsistenz der Schlagschäume kann in einem weiteren Bereich variiert werden. So ist es möglich, leichte fließfähige Schlagschäume oder aber steife, schnittfeste Schäume herzustellen. Ebenso kann man die mittlere Größe der Gasblasen, ihre Größenverteilung und ihre Anordnung in der Flüssigkeitsmatrix durch die Auswahl der Tenside, der Lösevermittler, Verdicker und Schaumstabilisatoren, Zelkeimbildner, der Temperatur und der Aufschlagtechnik in einem weiten Bereich variieren, so daß man in einfacher Weise Dichte, Offenzelligkeit oder wandstärke des Matrixmaterials einstellen kann. Die Temperaturen der polymerisierbaren wäßrigen Mischung liegen während des Schäumvorgangs in dem Bereich von -10 bis 100, vorzugsweise 0 bis +50°C. In jedem Falle werden bei der Schaumerzeugung Temperaturen angewandt, die unterhalb des Siedepunkts von Bestandteilen der polymerisierbaren wäßrigen Mischung liegen. Die Schaumerzeugung kann auch unter erhöhtem Druck erfolgen, z.B. bei 1,5 bis 25 bar. Bevorzugt wird jedoch unter Atmosphärendruck gearbeitet.

Gegenüber den bisher bekannten Verfahren zur Herstellung von schaumförmigen Superabsorbern ist ein wesentlicher Vorteil der erfindungsgemäßen Herstellung solcher Schäume darin zu sehen, daß man in der ersten Verfahrensstufe des erfindungsgemäßen Verfahrens geschäumte, polymerisierbare wäßrige Mischungen erhält, die über einen längeren Zeitraum, z.B. bis zu 6 Stunden stabil sind, so daß sie beispielsweise problemlos gehandhabt werden können. Die noch nicht polymerisierten schaumförmigen Mischungen können beispielsweise für die nachfolgende Polymerisation in eine geeignete Form gebracht werden, um die für eine bestimmte Anwendung gewünschten Formkörper herzustellen. Der bei der Formgebung der geschäumten polymerisierbaren wäßrigen Mischung möglicherweise anfallende Abfallschaum kann ohne weiteres in den Prozeß zurückgeführt werden. Das geschäumte polymerisierbare Material kann beispielsweise in der gewünschten Stärke auf ein temporäres Trägermaterial, das vorteilhafterweise mit einer Antihaftbeschichtung ausgestattet ist, aufgetragen werden. Man kann beispielsweise den Schaum auf eine Unterlage aufrakeln. Eine andere Möglichkeit besteht darin, die polymerisierbare schaumförmige wäßrige Mischung in Formen einzufüllen, die ebenfalls antihaftbeschichtet sind und den Schaum darin auszupolymerisieren.

Da die geschäumte polymerisierbare wäßrige Mischung eine lange Standzeit aufweist, eignet sich diese Mischung auch für die Herstellung von Verbundmaterialien. So kann beispielsweise der nach der Schlagschaumerzeugung hergestellte polymerisierbare Schaum auf ein permanentes Trägermaterial aufgebracht werden, z.B. Folien aus Polymeren (z.B. Folien aus Polyethylen, Polypropylen oder Polyamid) oder Metallen, Vliesen, Fluff, Tissues, Gewebe, natürliche oder synthetische Fasern, oder auf andere Schäume. Bei der Herstellung von Verbundmaterialien kann es unter Umständen auch vorteilhaft sein, den polymerisierbaren Schaum in Gestalt von bestimmten Strukturen oder in unterschiedlicher Schichtdicke auf ein Trägermaterial aufzubringen. Es ist jedoch auch möglich, den polymerisierbaren Schaum auf Fluff-Schichten aufzutragen und sie so zu imprägnieren, daß der Fluff nach der Polymerisation integraler Bestandteil des Schaums ist. Die in der ersten Verfahrensstufe erhältliche geschäumte polymerisierbare wäßrige Mischung kann auch zu großen Blöcken geformt und polymerisiert werden. Die Blöcke können nach der Polymerisation zu kleineren Formkörpern geschnitten oder gesägt werden. Man kann auch sandwichartige Strukturen herstellen, indem man eine geschäumte polymerisierbare wäßrige Mischung auf eine Unterlage aufträgt, die schaumförmige Schicht mit einer Folie, Vliesen, Tissues, Geweben, Fasern oder anderen Schäumen gegebenenfalls aus einem anderen Material als die zunächst verwendete Unterlage abdeckt und wiederum Schaum aufträgt und gegebenenfalls mit einer weiteren Folie, Vliesen, Tissues, Geweben, Fasern oder anderen Schäumen abdeckt. Der Verbund wird dann in der zweiten Verfahrensstufe der Polymerisation unterworfen. Man kann jedoch auch sandwichartige Strukturen mit weiteren Schaumschichten herstellen.

In der zweiten Stufe des Verfahrens zur Herstellung der erfindungsgemäßen superabsorbierenden Schäume erfolgt die Polymerisation der geschäumten polymerisierbaren wäßrigen Mischung. Die Polymerisation kann je nach verwendetem Initiator durch Temperaturerhöhung, durch Lichteinwirkung, durch Bestrahlen mit Elektronenstrahlen oder auch durch Temperaturerhöhung und Lichteinwirkung erfolgen. Um die Temperatur der geschäumten polymerisierbaren wäßrigen Mischung zu erhöhen, kann man alle in der Technik üblichen Verfahren anwenden, beispielsweise den Schaum mit heizbaren Platten in Kontakt bringen, Einwirkung von Infrarotbestrahlung auf den polymerisierbaren Schaum oder Beheizen mit Hilfe von Mikrowellen. Erfindungsgemäße Schaumschichten mit einer Schichtdicke von bis zu etwa 1 Millimeter stellt man beispielsweise durch einseitiges Erwärmen oder insbesondere durch einseitiges Bestrahlen her. Falls dickere Schichten eines Schaums hergestellt werden sollen, z.B. Schäume mit Dicken von mehreren Zentimetern, ist die Erwärmung des polymerisierbaren geschäumten Materials mit Hilfe einer Mikrowelle besonders vorteilhaft, weil auf diesem Wege eine relativ gleichmäßige Erwärmung erreicht werden kann. Die Polymerisation erfolgt dabei beispielsweise bei Temperaturen von 20 bis 180, vorzugsweise in dem Bereich von 20 bis 100°C.

Schaumschichten von mittleren Schichtdicken, d.h. mit einer Dicke im Bereich von etwa 1 Millimeter bis etwa 2 Zentimeter, wie z.B. von etwa 2 Millimeter bis etwa 1 Zentimeter, stellt man vorzugsweise in folgender Weise her: Anstatt die Polymerisation nur einflächig zu initiieren, bewirkt man eine beidflächige Initiierung, indem man eine Schicht der erfindungsgemäß geschäumten Masse einer beidflächigen Wärmebehandlung und/oder Bestrahlung mit Licht aussetzt. Die Behandlung beider Flächen der Schaumschicht kann erfindungsgemäß synchron oder in beliebiger zeitlicher Reihenfolge asynchron oder zeitlich versetzt erfolgen. Man kann beispielsweise die Wärmebehandlung beider Teilflächen einer Schaumschicht gleichzeitig oder zeitlich versetzt einmalig oder mehrmals pro Teilfläche durchführen. Ebenso kann man bei der Bestrahlung mit Licht verfahren. Es besteht aber auch die Möglichkeit, jede Teilfläche sowohl mit Wärme als auch mit Licht zu behandeln, wobei Wärme und Licht gleichzeitig oder in beliebiger Abfolge, einmalig oder mehrfach auf die gleiche Teilfläche der Schaumschicht einwirken können. Am zweckmäßigsten ist jedoch gewöhnlich die einmalige Anwendung von Wärme und/oder Licht je Teilfläche der Schaumschicht.

Da die Wärmebehandlung zweckmäßigerweise durch Kontaktheizung erfolgt und das dafür verwendete Trägermaterial gewöhnlich lichtundurchlässig ist, wird die beidseitige Polymerisationsinitiation am zweckmäßigsten durch Kontaktheizen einer Teilfläche und, beispielsweise simultanes, Bestrahlen der gegenüberliegenden Teilfläche durchgeführt. Diese Verfahrensvariante sowie das beidseitige Kontaktheizen eignen sich insbesondere zur Herstellung von Verbundmaterialien.

Die Wärmebehandlung erfolgt bei der beidflächigen Polymerisationsinitiation gewöhnlich in einem Bereich von etwa 50 bis etwa 200°C, vorzugsweise bei etwa 80 bis etwa 160°C. Typische Kontaktzeiten liegen dabei bei etwa 0,5 bis etwa 25 Minuten je Teilfläche der Schaumschicht, vorzugsweise bei etwa 2 bis etwa 15 Minuten. Zur Bestrahlung verwendet man vorzugsweise Licht aus dem UV/VIS-Bereich, d.h. Licht aus dem ultravioletten oder sichtbaren Bereich des Spektrums, wie z.B. Licht mit einer Wellenlänge im Bereich von etwa größer 200 nm bis etwa 750 nm, beispielsweise etwa 250 nm bis etwa 700 nm, wie etwa UV-A-Strahlung der Wellenlänge 315 bis 400 nm. Die Dauer der Bestrahlung kann ebenfalls im Bereich von etwa 0,1 bis etwa 25 Minuten, vorzugsweise bei etwa 0,5 bis 10 Minuten, je Teilfläche der Schaumschicht liegen.

Bei kombinierter Wärmebehandlung und Bestrahlung derselben oder gegenüberliegender Teilflächen der Schaumschicht können die jeweilige Dauer von Wärmebehandlung und Bestrahlung gleich oder verschieden sein. In Abhängigkeit von Zusammensetzung und Dicke der Schaumschicht, Art und Menge der verwendeten Polymerisationsinitiatoren, Intensität und Wellenlänge des Lichts sowie Temperatur der Kontaktheizvorrichtung und anderer Kriterien kann es aber von Vorteil sein, Wärmebehandlung und Bestrahlung über unterschiedlich lange Zeitintervalle durchzuführen. Die gewählten Zeitintervalle können z.B. zeitlich aufeinander folgen. Beispielsweise kann auf eine z.B. 3-minütige Erwärmung der ersten Teilfläche eine z.B. 2-minütige Bestrahlung der gegenüberliegenden zweiten Teilfläche folgen. Daran kann sich gegebenenfalls eine z.B. 2-minütige Wärmebehandlung der ersten und/oder der zweiten Teilfläche anschließen. Diesen Behandlungsrhythmus kann man gegebenenfalls unter Beibehaltung oder Veränderung der gewählten Zeitintervalle einmal oder mehrfach wiederholen. Die gewählten Zeitintervalle können sich aber auch überlappen. Beispielsweise kann man dabei die Bestrahlung nur über einen Teil des Wärmebehandlungsintervalls aufrechterhalten. So kann man beispielsweise die erste Teilfläche der Schaumschicht z.B. 2 Minuten erhitzen und anschließend z.B. weitere 4 Minuten erhitzen und synchron dazu die gegenüberliegende Fläche 4 Minuten bestrahlen. Ebenso ist es vorstellbar, die beiden Teilflächen zunächst z.B. 3 Minuten synchron zu erhitzen bzw. zu bestrahlen und anschließend die Wärmebehandlung der einen Teilfläche z.B. 2 Minuten fortzusetzen, nachdem man die Bestrahlung der anderen Teilfläche beendet hat. Auch diese Behandlungsrhythmen kann man gegebenenfalls unter Beibehaltung oder Veränderung der gewählten Zeitintervalle einmal oder mehrfach wiederholen.

Bei Initiierung der Polymerisation durch Lichteinwirkung auf das geschäumte polymerisierbare Material kann man alle konventionellen Belichtersysteme anwenden, sofern ihr Emissionsspektrum an den eingesetzten Photoinitiator adaptiert ist. Bei einem Start der Polymerisation durch Belichten wird vorteilhaft eine Kombination eines Photoinitiators eines thermischen Initiators oder/und aber ein Photoinitator eingesetzt, der auch als thermischer Initiator wirken kann, z.B. Azoinitiatoren. Da sich der Schaum während der Polymerisation durch die hohe Polymerisationswärme stark erwärmt, wird auf diese Weise ein besonders schneller und effektiver Ablauf der Polymerisationsreaktion erreicht. Bei der Initiierung durch Lichteinwirkung liegt die Polymerisationstemperatur in dem Bereich von 0 bis 150, vorzugsweise 10 bis 100°C.

Ein wesentlicher Vorteil des erfindungsgemäßen Verfahrens ist darin zu sehen, daß die Polymerisation unter weitgehendem Erhalt der Struktur der geschäumten polymerisierbaren wäßrigen Mischung abläuft, d.h. der polymerisierbare Schaum verändert während der Polymerisation sein Volumen nur unwesentlich. Die Polymerisationsreaktion wird durch die Starttemperatur, die Initiierungstechnik oder die Wärmeabfuhr beeinflußt. Die Polymerisationstemperatur wird vorzugsweise dahingehend kontrolliert, daß ein Sieden der polymerisierbaren wäßrigen Mischung vermieden wird. Mit fortschreitender Polymerisation tritt eine Verfestigung des Schaums infolge zunehmender Gelbildung ein. Der Schaum hat zumindest teilweise eine offenzellige Struktur. Vorzugsweise ist er zu mehr als 75 % offenzellig.

Nach der Polymerisation haben die vorliegenden Schäume einen Wassergehalt zwischen 10 % und 80 %. Im Prinzip können durch den Einsatz von Alkanolaminen Schäume erhalten werden, die auch im getrockneten Zustand flexibel sind. Da die Schäume jedoch hygroskopisch sind und aus der Luft ohnehin Feuchtigkeit aufnehmen, ist es sinnvoll eine Restfeuchte im Bereich von 1 - 20, bevorzugt 5 - 15 Gew.-% im Schaum zu belassen. Ferner kann es je nach Zusammensetzung des Schaums und dem beabsichtigten Einsatzgebiet auch sinnvoll sein einen davon abweichenden Feuchtegehalt im Schaum zu belassen.

Der Schaum kann mit Hilfe konventioneller Techniken getrocknet werden, beispielsweise durch Erhitzen mit einem heißen Gasstrom, durch Anlegen von Vakuum, durch Infrarotbestrahlung oder durch Erhitzen mit Mikrowellenstrahlung. Die Mikrowellenstrahlung erweist sich auch hier wiederum beim Trocknen von großvolumigen Formkörpern als vorteilhaft. Bei der Trocknung sollte die Temperatur kleiner 180°C, bevorzugt kleiner 120°C liegen. Es kann vorteilhaft sein mit einem Gasstrom zu trocknen, der einen definierten Feuchtegehalt aufweist (bis hin zum Einsatz von Wasserdampf), so daß auf diesem Weg der Schaum nur bis zu einem definierten Feuchtegehalt getrocknet wird.

Nach dem erfindungsgemäßen Verfahren erhält man einen überwiegend offenzelligen Superabsorberschaum, der vorzugsweise zu mindestens 75 % offenzellig ist.

Wie oben bereits angegeben wurde, kann eine inhomogene Vernetzungsdichte bei den erfindungsgemäßen Superabsorberschäumen bereits während der Herstellung erzeugt werden. Dies ist besonders vorteilhaft, wenn man als Monomere der oben beschriebenen Komponenten

    (a) Acrylsäure, Methacrylsäure, Vinylsulfonsäure, Acrylamidopropansulfonsäure oder deren Mischungen und
    (c) eine Mischung aus mindestens einem wasserlöslichen und mindestens einem wasserunlöslichen Vernetzer einsetzt.

Dennoch kann es wünschenswert sein, den Vernetzungsgrad des Schaumes nachträglich zu verändern. Um dieses Ziel zu erreichen, kann man beispielsweise während der Polymerisation durch den Zusatz geeigneter Monomere in das Gel latente Vernetzungsstellen einbauen, die unter den Bedingungen der Schaumherstellung nicht zu Vernetzungsreaktionen führen, jedoch unter speziellen Bedingungen, die nachträglich angewandt werden können, z.B. durch stark erhöhte Temperatur, in der Lage sind, weitere Vernetzungspunkte in der Gelstruktur zu bilden. Als Beispiele für solche Monomere kann der Einbau von Hydroxygruppen enthaltenden Verbindungen dienen, die bei höherer Temperatur, d.h. bei Temperaturen oberhalb von 150°C in der Lage sind, mit den Carboxylgruppen in der Schaumstruktur zu reagieren. Geeignete Verbindungen, die latente Vernetzungsstellen aufweisen, sind beispielsweise Hydroxyethylacrylat, Hydroxypropylacrylat, Hydroxybutylacrylat, Monoacrylsäureester des Glycerins, Monoacrylate oder Monomethacrylate von Polyethylenglykolen mit mindestens 2 Ethylenglykoleinheiten, Monoacrylate oder Monomethacrylate von Polypropylenglykolen mit mindestens 2 Propylenglykoleinheiten und Monomethacrylate von mehrwertigen Alkoholen, z.B. Hydroxybutylmethacrylat, Hydroxypropylmethacrylat, Hydroxyethylmethacrylat oder Glycerinmonomethacrylat.

Als weitere Möglichkeit einer homogenen Nachvernetzung bietet sich der nachträgliche Zusatz von Vernetzungsreagenzien an, d.h. Verbindungen, die mindestens zwei reaktive Gruppen aufweisen, die unter geeigneten Bedingungen in der Lage sind, z.B. beim Erhitzen auf Temperaturen von mindestens 70°C, mit den Säuregruppen des schaumförmigen Hydrogel zu reagieren. In diesem Falle ist es auch möglich, gesteuert über die Eindringtiefe des Vernetzers, eine Modifikation der inhomogenen Vernetzungsdichte zu erreichen. Geeignete Vernetzer bilden mit den Carboxylgruppen der Polymermatrix kovalente oder ionische Bindungen. Geeignete Vernetzungsmittel sind Verbindungen, die mindestens zwei funktionelle Gruppen der gleichen oder unterschiedlicher Art aufweisen, z.B. Hydroxy-, Amino-, quaternäre Ammonium-, Isocyanato-, Epoxi-, Aziridino-, Ester- oder Amidgruppen. Bevorzugte Nachvernetzungsmittel sind Polyalkohole wie Glycerin, Butylenglykol, Propylenglykol oder Bisepoxide. Mit solchen Vernetzern kann die Reaktion z.B. in den Temperaturbereich von 70 - 170, vorzugsweise bei 100 - 160°C erfolgen. Das Auftragen der Vernetzungsmittel auf das geschäumte Material kann beispielsweise durch Sprühen, Tauchen oder durch Gasphasenabscheidung erfolgen.

Erfindungsgemäß kann man den Schaum in der gewohnten Weise aber mit einem geringeren Neutralisationsgrad, typischerweise zwischen 0 und 60 %, bevorzugt 15 bis 40 %, als endgültig beabsichtigt herstellen und mindestens ein Alkanolamin nachträglich aufbringen z.B. durch Aufsprühen der Alkanolamine, deren Lösungen in Lösemitteln oder Lösemittelgemischen. Als Lösemittel für Alkanolamine können z.B. Verwendung finden: Wasser, Methanol, Ethanol, isoPropanol, Aceton. Bevorzugt ist Wasser. Die Nachneutralisation erfolgt zweckmäßigerweise nach der Polymerisation und vor der Trocknung. Es ist aber auch möglich, mindestens ein Alkanolamin zu einem späteren Zeitpunkt im Prozeßablauf auf das schaumförmige Hydrogel aufzubringen.

Für die Anwendung von sekundären bzw. primären Alkanolaminen ist diese Vorgehensweise zwingend. Tertiäre Alkanolamine können - wie oben bereits beschrieben - zur Neutralisation der Monomeren (a) eingesetzt werden und außerdem ebenso wie primäre, sekundäre und quaternäre Alkanolamine zur Neutralisation der Säuregruppen des schaumförmigen Hydrogels nach dem Polymerisieren verwendet werden. In manchen Fällen hat sich eine Arbeitsweise als vorteilhaft herausgestellt, bei der man die Säuregruppen enthaltenden Monomeren (a) zunächst partiell mit einem tertiären Alkanolamin neutralisiert (z.B. zu 20 bis 50 Mol-%), dann polymerisiert und im Anschluß daran die restlichen freien Säuregruppen des schaumförmigen Hydrogels mit einem Alkanolamin, vorzugsweise einem primären Alkanolamin wie Ethanolamin, neutralisiert, wobei der Gesamtneutralisacionsgrad der Säuregruppen im Hydrogel 55 bis 95, vorzugsweise 65 bis 85 Mol-% beträgt.

Die verwendeten Alkanolamine können in ihrer Struktur primär, sekundär, tertiär oder quaternär sein und einwertige, mehrwertige oder polyfunktionelle Basen darstellen. Die Alkanolamine können zusätzlich zu ihren Amino- und Hydroxylgruppen weitere funktionelle Gruppen wie z.B. Ester, Urethan, Ether, Thioether, Harnstoff usw. tragen. Eingesetzt werden können z.B. niedermolekulare Verbindungen wie Triethanolamin, Methyldiethanolamin, Dimethylethanolamin, Ethanolamin, N-Hydroxyethylmorpholin, Dimethylaminodiglycol, N,N,N',N'-Tetra-(hydroxyethyl)-ethylendiamin, N,N,N',N'-Tetra-(hydroxypropyl)-ethylendiamin, Dimethylaminotriglycol, Diethylaminoethanol, 3-Dimethylamino-1,2-propandiol, Triisopropanolamin, Diisopropylaminoethanol, Cholinhydroxid, Cholincarbonat, 2-tert.-Butylaminoethanol, Tris(oxymethyl)aminomethan, 3-Amino-1-prooanol, Isopropanolamin, 2-(2-Aminoethoxy)ethanol, 2-Amino-2-methyl-1-propanol oder aber oligomere oder Polymere wie z.B. mit Ethylenoxid, Propylenoxid, Glycidol oder anderen Epoxiden umgesetzte Aminogruppen-tragende Polymerisate oder Kondensate wie z.B. Polyethylenimine oder Polyvinylamine, Umsetzungsprodukte aus mindestens bifunktionellen, niedermolekularen Alkanolaminen mit mindestens bifunktionellen Reagenzien, die in der Lage sind, entweder mit der Hydroxyl- oder der Aminogruppe der Alkanolamine zu reagieren, wie z.B. Carbonsäuren, Estern, Epoxiden, Isocyanaten.

Vorzugsweise kommen Triethanolamin, Methyldiethanolamin, Dimethylaminodiglycol, Dimethylethanolamin, Ethanolamin, N,N,N',N'-Tetra- (hydroxyethyl) -ethylendiamin in Betracht.

Die erhaltenen Alkanolamin-haltigen Schäume sind klebrig. Die Klebrigkeit kann durch Puderung mit feinteiligen Pulvern vollständig beseitigt werden. Im Prinzip sind alle organischen oder anorganische Materialien in feiner Pulverform geeignet, sofern sie hydrophil sind, wie z.B. feinteiliges Siliziumoxid (Aerosil®), Silikate, Talkum, Guarkernmehl, Tarakernmehl, Johannisbrotkernmehl, alle Arten von Stärken, vernetzte oder nicht vernetzte Polyacrylsäuren oder deren Salze, Polyvinylalkohole, Copolymere der Maleinsäure, Titandioxid, Zeolithe, Cellulose, Carboxymethylcellulose und Hydroxyechylcellulose. Bevorzugt sind nicht wasserlösliche Materialien, insbesondere Talkum und Aerosil®. Die Puderung erfolgt zweckmäßigerweise nach der Polymerisation, kann aber auch zu jedem beliebigen späteren Zeitpunkt im Herstellprozeß durchgeführt werden. Die Aufwandmengen liegen zwischen 0,01 und 10 %, bevorzugt zwischen 0,1 und 5 %, bezogen auf das Gewicht des Schaums.

Die erfindungsgemäßen Superabsorberschäume haben beispielsweise eine Dichte von $10^{-3}$ bis 0,9, vorzugsweise 0,05 bis 0,7 g/cm$^3$. Die Dichte von Superabsorberschäumen wird gravimetrisch bestimmt. Aus einer gleichmäßigen Schaumschicht mit einer definierten Dicke zwischen 3 und 5 mm schneidet man beispielsweise mit einem scharfen Messer Quadrate mit einer Seitenlänge von 5 cm aus. Diese Proben werden gewogen und das erhaltene Gewicht durch das aus den Maßen errechnete Volumen dividiert.

Die Aufnahmekapazität des schaumförmigen Superabsorbers an Wasser pro Gramm Superabsorber wird an Schaumstücken bestimmt, die eine Dicke von 3 mm haben und jeweils 1 g wiegen. Die Prüfung der Retention erfolgt hierbei nach dem sogenannten Teebeuteltest. Als Flüssigkeit dient dabei eine 0,9 %ige Kochsalzlösung, 1 g des schaumförmigen Materials wird in einen Teebeutel gefüllt, der dann verschlossen wird. Dabei ist darauf zu achten, daß der Teebeutel genügend Raum zum vollständigen Ausquellen bietet. Der Teebeutel wird danach eine bestimmte Zeit lang in die Flüssigkeit eingetaucht und nach einer Abtropfdauer von 10 Minuten zurückgewogen. Für die Berechnung der Absorptionskapazität muß ein Blindversuch durchgeführt, werden, bei dem ein Teebeutel ohne schaumförmigen Superabsorber in die Lösung eingetaucht und das Gewicht des Teebeutels nach der oben angegebenen Abtropfdauer von 10 Minuten bestimmt wird. Die Aufnahmekapazität ergibt sich dann aus folgender Gleichung (1):

$$\text{Aufnahmekapazität} = \frac{\text{Gewicht des Teebeutels mit Superabsorberschaum} - \text{Gewicht des Teebeutels im Blindversuch}}{\text{Gewicht des eingewogenen Superabsorberschaums}} \quad (1)$$

Die Aufnahmegeschwindigkeit (Absorption Speed, im folgenden mit AS bezeichnet) wurde dadurch ermittelt, daß man aus gleichmäßig 3 mm dicken Schaumschichten rechteckige Proben mit einem Gewicht von 1 g mit Hilfe eines scharfen Messers ausschnitt. Diese Proben wurden in einer Petrischale mit 20 g synthetischem Urin übergossen. Mit Hilfe einer Stoppuhr wurde die Zeit ermittelt, die der Schaumstoff benötigte, um den synthetischen Urin vollständig aufzunehmen. Die Aufnahmegeschwindigkeit (AS) in g/g·sec errechnet sich aus folgender Gleichung (2):

$$AS = 20 \text{ g}/(1 \text{ g} * \text{ gemessene Zeit (in sec)}] \quad (2)$$

Rezeptur für synthetischen Urin:
in 1 l destilliertem Wasser werden die folgenden Salze gelöst:

2,00 g KC1

2,00 g $Na_2SO_4$
0,85 g $NH_4H_2PO_4$
0,15 g $(NH_4)_2HPO_4$
0,19 g $CaCl_2$
0,23 g $MgCl_2$

Die eingesetzten Salze müssen wasserfrei sein.

Bestimmung der Monomerschaumdichte:

Genau 100 ml des Monomerschaums werden in einen Meßzylinder eingefüllt und das Gewicht dieses Schaumvolumens bestimmt. Durch Division des ermittelten Gewichts in g durch 100 wird die Dichte in $g/cm^3$ erhalten.

Flexibilitätsbestimmung:

In Plexiglaskammern, Bola Standard-Exsikkator V1854-01, der Firma Bohlender, werden bei 20°C definierte Luftfeuchten dadurch eingestellt, daß in die Kammern gesättigte Lösungen von spezifischen Salzen, die in Kontakt mit einem ungelösten Bodenkörper sind, eingebracht werden. Verwendung finden die folgenden Salze:

15 % rel. Luftfeuchte    LiCl-Monohydrat
20 % rel. Luftfeuchte    Kaliumacetat
32 % rel. Luftfeuchte    $CaCl_2$-Hexahydrat

Von jedem Schaum werden ca. 2 cm x 2 cm große, getrocknete Stücke in diese Kammern eingebracht und die Veränderung des Feuchtegehalts in den Schäumen gravimetrisch verfolgt bis die Proben den Gleichgewichtszustand erreicht haben.
Danach wird die Flexibilität anhand einer vierstufigen Notenskala beurteilt:

flexibel          die Probe läßt sich leicht um 180°C biegen und fühlt sich weich an,
zähelastisch      die Probe läßt sich nur noch schwer um 180 biegen, bricht aber nicht,
kaum flexibel     die probe läßt sich noch biegen, bricht aber zwischen 90° und 180°,
hart              die Probe bricht schon bei einer Verbiegung von weniger als 90°,

Für die Flexibilitätsbestimmung bei erhöhter Temperatur wird ein Klimaprüfschrank der Firma Weiß, Typ 125 SG+10JU/70DU, eingesetzt, der ein gezieltes Einstellen von Temperatur und relativer Luftfeuchte gestattet.
Zur Bestimmung der Flexibilität bei tiefen Temperaturen werden Schaumproben durch Besprühen auf einen Wassergehalt von 10 % angefeuchtet. Dieser Wassergehalt entspricht der Gleichgewichtswasseraufnahme bei einer relativen Luftfeuchte von 20 %. Anschliessend werden die Proben in geschlossenen Polyethylentüten über Nacht auf die entsprechende Temperatur abgekühlt und deren Flexibilität bei der jeweils eingestellten Temperatur nach obigen Kriterien beurteilt.
Die oben beschriebenen wasserabsorbierenden, schaumförmigen, vernetzten Polymerisate können für sämtliche Zwecke verwendet werden, für die die in der Literatur beschriebenen schaumförmigen Superabsorber eingesetzt werden. Sie werden z.B. in Sanitärartikeln, die zur Adsorption von Körperflüssigkeiten eingesetzt werden und in Verbandmacerial zur Abdeckung von Wunden verwendet. Sie eignen sich beispielsweise als wasserabsorbierender Bestandteil in Windeln, Damenbinden und Inkontinenzartikeln. Sie können in Form von Verbundmaterialien eingesetzt werden. Schaumförmige Superabsorber können außerdem als Dichtungsmaterial, als Bodenverbesserungsmittel, als Bodenersatzstoff und als Verpackungsmaterial verwendet werden. Spezielle Ausgestaltungen von Gegenständen, die schaumförmige Superabsorber enthalten, werden beispielsweise ausführlich in der WO-A-94/22502 beschrieben. Die schaumförmigen Superabsorber eignen sich außerdem zur Entwässerung von Schlämmen, zum Eindicken wäßriger Lacke, z.B. für die Entsorgung von Restmengen nicht verbrauchter wäßriger Lacke oder Farben, indem man z.B. pulverförmige schaumförmige Superabsorber zu wäßrigen Lackresten zugibt, bis eine Verfestigung eintritt. Die schaumförmigen, wasserabsorbierenden, vernetzten Polymerisate können außerdem zur Entwässerung von wasserhaltigen Ölen verwendet werden. Sie können beispielsweise in Form eines Pulvers mit einem mittleren Teilchendurchmesser von 150 µm bis 5 mm bei den oben beschriebenen Anwendungen eingesetzt werden.
Die oben beschriebenen Schäume können aufgrund ihrer Eigenschaften verschiedene Funktionen in Hygieneartikeln bei der Speicherung von Körperflüssigkeiten erfüllen:

-    Akquisition

- Distribution und/oder
- Speicherung

Die Speicherung von Körperflüssigkeiten wird von den Schäumen vollständig übernommen, während für die Funktionen Akquisition und Distribution gegebenenfalls weitere Bestandteile wie high loft-Nonwovens, Polypropylen-Vliese, Polyester-Vliese oder chemisch modifizierte Zellstoffe unterstützend als Schicht auf den Schäumen Verwendung finden können.

Die Prozentangaben in den Beispielen bedeuten Gewichtsprozent, sofern aus dem Zusammenhang nichts anderes hervorgeht.

Beispiele

Die Flexibilität und die Absorptionseigenschaften der Schäume, die gemäß den Beispielen und Vergleichsbeispielen erhalten wurden, sind in den Tabellen 1 - 3 angegeben.

Beispiel 1

In einem verschlossenen Glas mit Schraubverschluß wurden mit Hilfe eines Magnetrührers die folgenden Komponenten vermischt:

| 105,39 | Acrylsäure (1,46 mol) |
|---|---|
| 158,03 | einer 37,3 %igen Natriumacrylatlösung in Wasser (0,63 mol) |
| 9,25 | Guarkernmehl |
| 1,85 | Polyethylenglykoldiacrylat von Polyethylenglykol der Molmasse 500 |
| 58,58 | einer 15 %igen, wäßrigen Lösung eines Additionsprodukts von 80 Mol Ethylenoxid an 1 mol eines linearen, gesättigten $C_{16}C_{18}$-Fettalkohols |
| 35,90 | Wasser |

Die erhaltene homogene Mischung wurde in einen geschlossenen 2 1-Kolben mit Kühlmantel und Tropftrichter eingefüllt, in den von unten Kohlendioxid eingeleitet, wurde. In den Kolben waren zwei Schneebesen der Firma BOKU eingesetzt, die über ein Getriebe mit einem Rührer RW28W der Firma IKA verbunden waren. Der Kohlendioxidstrom wurde so eingestellt, daß er mit einer Geschwindigkeit von 100 l/h durch die Reaktionsmischung perlte. Der Rührmotor wurde zunächst auf eine Drehzahl von 200 UpM eingestellt und für 20 min Kohlendioxid durch die Mischung geleitet um gelösten Sauerstoff zu entfernen. Während dieser Zeit wurden 140,26 g Triethanolamin (0,94 mol) unter Kühlen so zugetropft, daß eine Endtemperatur von 16°C erreicht wurde.

Anschließend wurden 4,63 g Pentan und 20,97 g einer 3 %igen Lösung von 2,2'-Azobis(2-amidinopropan) dihydrochlorid in Wasser zugegeben und die Rührerdrehzahl auf 735 UpM erhöht. Die Mischung wurde bei dieser Drehzahl für 3,5 min aufgeschlagen. Nach Ende der Aufschlagsperiode wurde ein feinzelliger, gut fließfähiger Schlagschaum erhalten.

Der erhaltene Monomerschaum wurde auf eine DIN-A3 große Glasplatte mit 3 mm hohen Rändern aufgebracht und mit einer zweiten Glasplatte bedeckt. Die Schaumprobe wurde synchron von beiden Seiten mit zwei UV/VIS-Strahler (UV 1000 der Firma Höhnle) für 4 Minuten bestrahlt.

Die erhaltene Schaumschicht wurde auf beiden Seiten mit ca. 0,3 g Talkum bepudert und in einem Vakuumtrockenschrank bei 70°C vollständig getrocknet. Zur Bestimmung der Eigenschaften wurde ein Teil des Schaums anschließend durch Besprühen mit Wasser auf eine Feuchte von 10 % eingestellt.

| Neutralisationsgrad | 75 mol-% |
|---|---|
| Monomerschaumdichte | 0,33 g/cm$^3$ |
| Polymerschaumdichte | 0,44 g/cm$^3$ |
| Griff | trocken, völlig klebfrei |

Beispiel 2

In einem verschlossenen Glas mit Schraubverschluß wurden mit Hilfe eines Magnetrührers die folgenden Komponenten vermischt:

| | |
|---|---|
| 52,68 g | Acrylsäure (0,73 mol) |
| 330,79 g | einer 40,0 %igen Triethanolammoniumacrylatlösung in Wasser (0,60 mol) |
| 9,25 g | Guarkernmehl |
| 1,85 g | Polyethylenglykoldiacrylat eines Polyethylenglykols der Molmasse 500 |
| 12,33 g | einer 15 %igen, wässrigen Lösung eines Additionsprodukts von 80 Mol Ethylenoxid an 1 mol eines linearen, gesättigten $C_{16}C_{18}$ Fettalkohols |
| 102,34 g | Wasser |

Die erhaltene homogene Mischung wurde in einen geschlossenen 2 1-Kolben mit Kühlmantel eingefüllt, in den von unten her Kohlendioxid eingeleitet wurde. In den Kolben waren zwei Schneebesen der Firma BOKU eingesetzt, die über ein Getriebe mit einem Rührer RW28 W der Firma IKA verbunden waren. Der Kohlendioxidstrom wurde so eingestellt, daß er mit einer Geschwindigkeit von 100 l/h durch die Reaktionsmischung perlt. Der Rührmotor wurde zunächst auf eine Drehzahl von 200 UpM eingestellt und für 20 min Kohlendioxid durch die Mischung geleitet, um gelösten Sauerstoff zu entfernen. Während dieser Zeit wurde die Innentemperatur mit Hilfe des Kühlmantels und eines Thermostaten auf 16°C abgekühlt.

Anschließend wurden 4,63 g Pentan und 20,97 g einer 3 %igen Lösung von 2,2'-Azobis(2-amidinopropan) dihydrochlorid in Wasser zugegeben und die Rührerdrehzahl auf 735 UpM erhöht. Die Mischung wurde bei dieser Drehzahl für 3,5 min aufgeschlagen. Nach Ende der Aufschlagsperiode wurde ein feinzelliger, gut fließfähiger Schlagschaum erhalten.

Der erhaltene Monomerschaum wurde auf eine DIN-A3 große Glasplatte mit 3 mm hohen Rändern aufgebracht und mit einer zweiten Glasplatte bedeckt. Anschließend wurde die Schaumprobe synchron von beiden Seiten mit zwei UV/VIS-Strahler (UV 1000 der Firma Höhnle) für 4 Minuten bestrahlt.

Die erhaltene Schaumschicht wurde auf beiden Seiten mit ca. 0,3 g Talkum bepudert und in einem Vakuumtrockenschrank bei 70°C vollständig getrocknet. Zur Bestimmung der Eigenschaften wurde ein Teil des Schaums anschließend durch Besprühen mit Wasser auf eine Feuchte von 10 % eingestellt.

| | |
|---|---|
| Neutralisationsgrad | 45,0 mol% |
| Monomerschaumdichte | 0,29 g/cm$^3$ |
| Polymerschaumdichte | 0,35 g/cm$^3$ |
| Griff | trocken, völlig klebfrei |

Beispiel 3

Auf die gleiche weise wie in Beispiel 1 wurde ausgehend von den nachstehenden Einsatzstoffen ein Schaum hergestellt:

| | |
|---|---|
| 127,96 g | Acrylsäure (1.78 mol) |
| 79,02 g | einer 37,3 %igen Natriumacrylatlösung in Wasser (0,31 mol) |
| 9,25 g | Guarkernmehl |
| 1,85 g | Polyethylenglykoldiacrylat eines Polyethylenglykols der Molmasse 400 |
| 58,58 g | einer 15 %igen, wässrigen Lösung eines Additionsprodukts von 80 Mol Ethylenoxid an 1 mol eines linearen, gesättigten $C_{16}C_{18}$ Fettalkohols |
| 45,58 g | Wasser |
| 187,01 g | Triethanolamin (1,25 mol) |
| 4,63 g | Pentan |
| 20,97 g | einer 3 %igen Lösung von 2,2'-Azobis(2-amidinopropan)dihydrochlorid in Wasser |

| | |
|---|---|
| Neutralisacionsgrad | 75 mol% |
| Monomerschaumdichte | 0,34 |
| Polymerschaumdichte | 0,48 |
| Griff | trocken, völlig klebfrei |

Beispiel 4

Auf die gleiche Weise wie in Beispiel 2 wurde ausgehend von den nachstehenden Einsatzstoffen ein Schaum hergestellt:

| | |
|---|---|
| 33,01 g | Acrylsäure (0,46 mol) |
| 379,98 g | einer 40,0 %igen Triethanolammoniumacrylatlösung in Wasser (0,69 mol) |
| 9,25 g | Guarkernmehl |
| 1,85 g | Polyethylenglykoldiacrylat eines Polyethylenglykols der Molmasse 600 |
| 12,33 g | einer 15 %igen, wässrigen Lösung eines Additionsprodukts von 80 Mol Ethylenoxid an 1 mol eines linearen, gesättigten $C_{16}C_{18}$ Fettalkohols |
| 72,83 g | Wasser |
| 4,63 g | Pentan |
| 20,97 g | einer 3 %igen Lösung von 2,2'-Azobis(2-amidinopropan)dihydrochlorid in Wasser |

| | |
|---|---|
| Neutralisationsgrad | 60 mol% |
| Monomerschaumdichte | 0,30 |
| Polymerschaumdichte | 0,40 |
| Griff | trocken, völlig klebfrei |

Beispiel 5

Auf die gleiche Weise wie in Beispiel 1 wurde ausgehend von den nachstehenden Einsatzstoffen ein Schaum hergestellt:

| | |
|---|---|
| 139,25 g | Acrylsäure (1,93 mol) |
| 39,51 g | einer 37,3 %igen Natriumacrylatlösung in Wasser (0,16 mol) |
| 9,25 g | Guarkernmehl |
| 1,85 g | Polyethylenglykoldiacrylat eines Polyethylenglykols der Molmasse 500 |
| 58,58 g | einer 15 %igen, wässrigen Lösung eines Additionsprodukts von 80 Mol Ethylenoxid an 1 mol eines linearen, gesättigten $C_{16}C_{18}$ Fettalkohols |
| 50,42 g | Wasser |
| 210,38 g | Triethanolamin (1,41 moi) |
| 4,63 g | Pentan |
| 20,97 g | einer 3 %igen Lösung von 2,2'-Azobis(2-amidinopropan)dihydrochlorid in Wasser |

| | |
|---|---|
| Neutralisationsgrad | 75 mol% |
| Monomerschaumdichte | 0,37 g/cm$^3$ |
| Polymerschaumdichte | 0,37 g/cm$^3$ |
| Griff | trocken, völlig klebfrei |

Beispiel 6

Auf die gleiche Weise wie in Beispiel 2 wurde ausgehend von den nachstehenden Einsatzstoffen ein Schaum hergescellt:

| | |
|---|---|
| 22,66 g | Acrylsäure (0,31 mol) |
| 405,84 g | einer 40,0 %igen Triethanolammoniumacrylatlösung in Wasser (0,73 mol) |
| 9,25 g | Guarkernmehl |
| 1,85 g | Polyethylenglykoldiacrylat eines Polyethylenglykols der Molmasse 650 |

(fortgesetzt)

| | |
|---|---|
| 12,33 | einer 15 %igen, wässrigen Lösung eines Additionsprodukts von 80 Mol Ethylenoxid an 1 mol eines linearen, gesättigten $C_{16}C_{18}$ Fettalkohols |
| 57,31 | Wasser |
| 4,63 | Pentan |
| 20,97 | einer 3 %igen Lösung von 2,2'-Azobis(2-amidinopropan)dihydrochlorid in Wasser |

| | |
|---|---|
| Neutralisationsgrad | 70 mol% |
| Monomerschaumdichte | 0,32 |
| Polymerschaumdichte | 0,50 |
| Griff | trocken, völlig klebfrei |

Beispiel 7

In einem verschlossenen Glas mit Schraubverschluß wurden mit Hilfe eines Magnetrührers die folgenden Komponenten vermischt:

| | |
|---|---|
| 79,89 | Acrylsäure (1,11 mol) |
| 262,77 | einer 40,0 %igen Triethanolammoniumacrylatlösung in Wasser (0,48 mol) |
| 9,25 | Guarkernmehl |
| 1,85 | Polyethylenglykoldiacrylat eines Polyethylenglykols der Molmasse 400 |
| 12,33 | einer 15 %igen, wässrigen Lösung eines Additionsprodukts von 80 Mol Ethylenoxid an 1 mol eines linearen, gesättigten $C_{16}C_{18}$ Fettalkohols |
| 143,16 | Wasser |

Die erhaltene homogene Mischung wurde in einen geschlossenen 2 1-Kolben mit Kühlmantel eingefüllt, in den von unten her Kohlendioxid eingeleitet wurde. In den Kolben waren zwei Schneebesen der Firma BOKU eingesetzt, die über ein Getriebe mit einem Rührer RW28 w der Firma IKA verbunden waren. Der Kohlendioxidstrom wurde so eingestellt, daß er mit einer Geschwindigkeit von 100 l/h durch die Reaktionsmischung perlte. Der Rührmotor wurde zunächst auf eine Drehzahl von 200 UpM eingestellt und für 20 min Kohlendioxid durch die Mischung geleitet, um gelösten Sauerstoff zu entfernen. Während dieser Zeit wurde die Innentemperatur mit Hilfe des Kühlmantels und eines Thermostaten auf 16°C abgekühlt.

Anschließend wurden 4,63 g Pentan und 20,97 g einer 3 %igen Lösung von 2,2'-Azobis(2-amidinopropan) dihydrochlorid in Wasser zugegeben und die Rührerdrehzahl auf 735 UpM erhöht. Die Mischung wurde bei dieser Drehzahl für 3,5 min aufgeschlagen. Nach Ende der Aufschlagsperiode wurde ein feinzelliger, gut fließfähiger Schlagschaum erhalten.

Der erhaltene Monomerschaum wurde auf eine Din-A3 große Glasplatte mit 3 mm höhen Rändern aufgebracht und mit einer zweiten Glasplatte bedeckt. Die Schaumprobe wurde synchron von beiden Seiten mit zwei UV/ VIS-Strahler (UV 1000 der Firma Höhnle) für 4 Minuten bestrahlt.

Die erhaltene Schaumschicht wurde mit einer 10 %igen wässrigen Triethanolaminlösung besprüht, so daß der Neutralisationsgrad von 30 mol% auf 75 mol% erhöht wurde und anschließend auf beiden Seiten mit ca. 0,3 g Talkum bepudert. Der Schaum wurde in einem Vakuumtrockenschrank bei 70°C vollständig getrocknet. Zur Bestimmung der Eigenschaften wurde ein Teil des Schaums anschließend durch Besprühen mit Wasser auf eine Feuchte von 10 % eingestellt.

| | |
|---|---|
| Neutralisationsgrad | 75 mol% |
| Monomerschaumdichte | 0,34 g/cm$^3$ |
| Polymerschaumdichte | 0,39 g/cm$^3$ |
| Griff | trocken, völlig klebfrei |

Beispiel 8

In einem verschlossenen Glas mit Schraubverschluß wurden mit Hilfe eines Magnetrührers die folgenden Komponenten vermischt:

| | |
|---|---|
| 112,86 | Acrylsäure (1,57 mol) |
| 131,68 | einer 37,3 %igen Natriumacrylatlösung in Wasser (0,52 mol) |
| 9,25 | Guarkernmehl |
| 1,85 | Polyethylenglykoldiacrylat eines Polyethylenglykols der Molmasse 500 |
| 37,00 | einer 15 %igen, wässrigen Lösung eines Additionsprodukts von 80 Mol Ethylenoxid an 1 mol eines linearen, gesättigten $C_{16}C_{18}$ Fettalkohols |
| 28,40 | Wasser |

Die erhaltene homogene Mischung wurde in einen geschlossenen 2 1-Kolben mit Kühlmantel und Tropftrichter eingefüllt, in den von unten her Stickstoff eingeleitet wurde. In den Kolben waren zwei Schneebesen der Firma BOKU eingesetzt, die über ein Getriebe mit einem Rührer RW28 W der Firma IKA verbunden waren. Der Stickstoffstrom wurde so eingestellt, daß er mit einer Geschwindigkeit von 100 l/h durch die Reaktionsmischung perlte. Der Rührmotor wurde zunächst auf eine Drehzahl von 200 UpM eingestellt und für 20 min Stickstoff durch die Mischung geleitet, um gelösten Sauerstoff zu entfernen. Während dieser Zeit wurden 124,53 g Methyldiethanolamin (1,05 mol) unter Kühlen so zugetropft, daß eine Endtemperatur von 16°C erreicht wurde.

Anschließend wurden 6,94 g Pentan und 20,97 g einer 3 %igen Lösung von 2,2'-Azobis(2-amidinopropan) dihydrochlorid in Wasser zugegeben und die Rührerdrehzahl auf 735 UpM erhöht. Die Mischung wurde bei dieser Drehzahl für 6 min aufgeschlagen. Nach Ende der Aufschlagperiode wurde ein feinzelliger, gut fließfähiger Schlagschaum erhalten.

Der erhaltene Monomerschaum wurde in eine 20 cm x 20 cm große, teflonbeschichtete Aluminiumform mit einem 3 mm hohen Rand gefüllt und mit einer Glasplatte bedeckt. Die Form wurde für 2 min auf eine Heizplatte (Ceran 500) mit einer Oberflächentemperatur von 115°C gestellt, anschließend für 2 min gleichzeitig von oben mit einem UV/ VIS-Strahler (UV 2000 der Firma Höhnle) belichtet und von unten weiter beheizt und schließlich für 2 min ohne Belichten auf der Heizplatte belassen.

Die erhaltene Schaumschicht wurde auf beiden Seiten mit ca. 0,3 g Talkum bepudert und in einem Vakuumtrockenschrank bei 70°C vollständig getrocknet. Zur Bestimmung der Eigenschaften wurde ein Teil des Schaums anschließend durch Besprühen mit Wasser auf eine Feuchte von 10 % eingestellt.

| | |
|---|---|
| Neutralisationsgrad | 75 mol% |
| Monomerschaumdichte | 0,36 g/cm$^3$ |
| Polymerschaumdichte | 0,43 g/cm$^3$ |
| Griff | trocken, völlig klebfrei |

Beispiel 9

Auf die gleiche Weise wie in Beispiel 1 wurde ausgehend von den nachstehenden Einsatzstoffen ein Schaum hergestellt:

| | |
|---|---|
| 120,18 | Acrylsäure (1,67 mol) |
| 105,13 | einer 37,3 %igen Natriumacrylatlösung in Wasser (0,42 mol) |
| 12,60 | Guarkernmehl |
| 8,82 | Polyethylenglykoldiacrylat eines Polyethylenglykols dder Molmasse 600 |
| 58,78 | einer 15 %igen, wässrigen Lösung eines Additionsprodukts von 80 Mol Ethylenoxid an 1 mol eines linearen, gesättigten $C_{16}C_{18}$ Fettalkohols |
| 55,99 | Wasser |
| 155,51 | Triethanolamin (1,04 mol) |
| 4,63 | Pentan |
| 20,99 | einer 3 %igen Lösung von 2,2'-Azobis(2-amidinopropan)dihydrochlorid in Wasser |

Nach dem Trocknen wurde der Schaum durch Besprühen mit Wasser auf einen Feuchtegehalt von 10 % eingestellt.

| | |
|---|---|
| Neutralisationsgrad | 70 mol% |

(fortgesetzt)

| | |
|---|---|
| Monomerschaumdichte | 0,26 g/cm$^3$ |
| Polymerschaumdichte | 0,26 g/cm$^3$ |
| Griff | trocken, völlig klebfrei |

Beispiel 10

Auf die gleiche Weise wie in Beispiel 1 wurde ausgehend von den nachstehenden Einsatzstoffen ein Schaum hergestellt:

| | |
|---|---|
| 127,93 g | Acrylsäure (1,77 mol) |
| 93,43 g | einer 37,3 %igen Natriumacrylatlösung in Wasser (0,37 mol) |
| 12,60 g | Guarkernmehl |
| 6,29 g | Polyethylenglykoldiacrylat eines Polyethylenglykols der Molmasse 400 |
| 58.78 g | einer 15 %igen, wässrigen Lösung eines Additionsprodukts von 80 Mol Ethylenoxid an 1 mol eines linearen, gesättigten $C_{16}C_{18}$ Fettalkohols |
| 63,02 g | Wasser |
| 152,42 g | Triethanolamin (1,02 mol) |
| 4,63 g | Pentan |
| 20,99 g | einer 3 %igen Lösung von 2,2'-Azobis(2-amidinopropan)dihydrochlorid in Wasser |

Nach dem Trocknen wurde der Schaum durch Besprühen mit Wasser auf einen Feuchtegehalt von 10 % eingestellt.

| | |
|---|---|
| Neutralisationsgrad | 65 mol% |
| Monomerschaumdichte | 0,28 g/cm$^3$ |
| Polymerschaumdichte | 0,23 g/cm$^3$ |
| Griff | trocken, völlig klebfrei |

Beispiel 11

Auf die gleiche Weise wie in Beispiel 1 wurde ausgehend von den nachstehenden Einsatzstoffen ein Schaum hergestellt:

| | |
|---|---|
| 119,27 g | Acrylsäure (1,66 mol) |
| 46,37 g | einer 37,3 %igen Natriumacrylatlösung in Wasser (0,18 mol) |
| 12,60 g | Guarkernmehl |
| 8,82 g | Polyethylenglykoldiacrylat eines Polyethylenglykols der Molmasse 400 |
| 58,78 g | einer 15 %igen, wässrigen Lösung eines Additionsprodukts von 80 Mol Ethylenoxid an 1 mol eines linearen, gesättigten $C_{16}C_{18}$ Fettalkohols |
| 92,84 g | Wasser |
| 178,34 g | Triethanolamin (1,20 mol) |
| 4,63 g | Pentan |
| 20,99 g | einer 3 %igen Lösung von 2,2'-Azobis(2-amidinopropan)dihydrochlorid in Wasser |

Nach dem Trocknen wurde der Schaum durch Besprühen mit Wasser auf einen Feuchtegehalt von 10 % eingestellt.

| | |
|---|---|
| Neutralisationsgrad | 75 mol% |
| Monomerschaumdichte | 0,27 g/cm$^3$ |
| Polymerschaumdichte | 0,25 g/cm$^3$ |
| Griff | trocken, völlig klebfrei |

Beispiel 12

Auf die gleiche Weise wie in Beispiel 1 wurde ausgehend von den nachstehenden Einsatzstoffen ein Schaum hergestellt:

| | |
|---|---|
| 140,44 | Acrylsäure (1,95 mol) |
| 12,60 | Guarkernmehl |
| 8,81 | Polyethylenglykoldiacrylat eines Polyethylenglykols der Molmasse 600 |
| 58,78 | einer 15 %igen, wässrigen Lösung eines Additionsprodukts von 80 Mol Ethylenoxid an 1 mol eines linearen, gesättigten $C_{16}C_{18}$ Fettalkohols |
| 121,60 | Wasser |
| 174,46 | Triethanolamin (1,17 mol) |
| 4,63 | Pentan |
| 20,99 | einer 3 %igen Lösung von 2,2'-Azobis(2-amidinopropan)dihydrochlorid in Wasser |

Nach dem Trocknen wurde der Schaum durch Besprühen mit Wasser auf einen Feuchtegehalt von 10 % eingestellt.

| | |
|---|---|
| Neutralisationsgrad | 60 mol% |
| Monomerschaumdichte | 0,27 g/cm$^3$ |
| Polymerschaumdichte | 0,23 g/cm$^3$ |
| Griff | trocken, völlig klebfrei |

Vergleichsbeispiel 1

In einem verschlossenen Glas mit Schraubverschluß wurden mit Hilfe eines Magnetrührers die folgenden Komponenten vermischt:

| | |
|---|---|
| 37,64 | Acrylsäure (0,52 mol) |
| 395,08 | einer 37,3 %igen Natriumacrylatlösung in Wasser (1,57) |
| 9,25 | Guarkernmehl |
| 1,85 | Polyethylenglykoldiacrylat eines Polyethylenglykols der Molmasse 400 |
| 58,58 | einer 15 %igen, wässrigen Lösung eines Additionsprodukts von 80 Mol Ethylenoxid an 1 mol eines linearen, gesättigten $C_{16}C_{18}$ Fettalkohols |
| 6,85 | Wasser |

Die erhaltene homogene Mischung wurde in einen geschlossenen 2 1-Kolben mit Kühlmantel eingefüllt, in den von unten'her Kohlendioxid eingeleitet wurde. In den Kolben waren zwei Schneebesen der Firma BOKU eingesetzt, die über ein Getriebe mit einem Rührer RW28 W der Firma IKA verbunden waren. Der Kohlendioxidstrom wurde so eingestellt, daß er mit einer Geschwindigkeit von 100 l/h durch die Reaktionsmischung perlte. Der Rührmotor wurde zunächst auf eine Drehzahl von 200 UpM eingestellt und für 20 min Kohlendioxid durch die Mischung geleitet um gelösten Sauerstoff zu entfernen. Während dieser Zeit wurde die Innentemperatur mit Hilfe des Kühlmantels und eines Thermostaten auf 16°C eingestellt. Anschließend wurden 4,63 g Pentan und 20,97 g einer 3 %igen Lösung von 2,2'-Azobis(2-amidinopropan)dihydrochlorid in Wasser zugegeben und die Rührerdrehzahl auf 735 UpM eingestellt. Die Mischung wurde bei dieser Drehzahl für 3,5 min aufgeschlagen. Nach Ende der Aufschlagsperiode wurde ein feinzelliger, gut fließfähiger Schlagschaum erhalten.

Der Monomerschaum wurde auf eine DIN-A3 große Glasplatte mit 3 mm hohen Rändern aufgebracht und mit einer zweiten Glasplatte bedeckt. Die Schaumprobe wurde synchron von beiden Seiten mit zwei UV/VIS-Strahler (UV 1000 der Firma Höhnle) für 4 Minuten bestrahlt.

Die erhaltene Schaumschicht wurde in einem Vakuumtrockenschrank bei 70°C vollständig getrocknet und anschließend durch Besprühen mit Wasser auf einen Feuchtegehalt von 25 % eingestellt.

| | |
|---|---|
| Neutralisationsgrad | 75 mol% |
| Monomerschaumdichte | 0,31 g/cm$^3$ |

(fortgesetzt)

| Polymerschaumdichte | $0,32 \text{ g/cm}^3$ |
|---|---|
| Griff | feucht, kaum klebrig |

Vergleichsbeispiel 2

Nach der gleichen Vorgehensweise wie im Vergleichsbeispiel 1 wurde ausgehend von den folgenden Komponenten ein Schaum hergestellt:

| | |
|---|---|
| 83,51 | Acrylsäure (1,16 mol) |
| 292,28 | einer 37,3 %igen Natriumacrylatlösung in Wasser (1,16 mol) |
| 9,25 | Guarkernmehl |
| 1,85 | Polyethylenglykoldiacrylat eines Polyethylenglykols der Molmasse 500 |
| 58,58 | einer 15 %igen, wässrigen Lösung eines Additionsprodukts von 80 Mol Ethylenoxid an 1 mol eines linearen, gesättigten $C_{16}C_{18}$ Fettalkohols |
| 69,55 | Wasser |
| 4,63 | Pentan |
| 20,97 | einer 3 %igen Lösung von 2,2'-Azobis(2-amidinopropan)dihydrochlorid in Wasser |

| Neutralisationsgrad | 50 mol% |
|---|---|
| Monomerschaumdichte | $0,26 \text{ g/cm}^3$ |
| Polymerschaumdichte | $0,29 \text{ g/cm}^3$ |
| Griff | feucht, kaum klebrig |

Vergleichsbeispiel 3

Nach der gleichen Vorgehensweise wie im Vergleichsbeispiel 1 wurde ausgehend von den folgenden Komponenten ein Schaum hergestellt:

| | |
|---|---|
| 116,90 | Acrylsäure (1,62 mol) |
| 175,37 | einer 37,3 %igen Natriumacrylatlösung in Wasser (0,70 mol) |
| 9,25 | Guarkernmehl |
| 6,48 | Polyethylenglykoldiacrylat eines Polyechylenglykols der Molmasse 500 |
| 58,58 | einer 15 %igen, wässrigen Lösung eines Additionsprodukts von 80 Mol Ethylenoxid an 1 mol eines linearen, gesättigten $C_{16}C_{18}$ Fettalkohols |
| 144,79 | Wasser |
| 4,63 | Pentan |
| 20,97 | einer 3 %igen Lösung von 2,2'-Azobis(2-amidinopropan)dihydrochlorid in Wasser |

| Neutralisationsgrad | 30 mol% |
|---|---|
| Monomerschaumdichte | $0,26 \text{ g/cm}^3$ |
| Polymerschaumdichte | $0,24 \text{ g/cm}^3$ |
| Griff | feucht, kaum klebrig |

Vergleichsbeispiel 4 (entsprechend JP-A-08073507)

In einem geschlossenen Kolben mit Rührer und Stickstoffeinleitung von unten wurde aus den folgenden Komponenten eine Mischung hergestellt:

| | |
|---|---|
| 180,00 | Acrylsäure (2,50 mol) |
| 74,50 | Triethanolamin (0,50 mol) |

(fortgesetzt)

| | |
|---|---|
| 42,00 | KOH (0,50 mol) |
| 74,00 | Wasser |
| 0,25 | Trimethylolpropantriacrylat |
| 3,33 | einer 15 %igen, wässrigen Natriumperoxodisulfatlösung |

Durch die Mischung wurde für 20 min ein Stickstoffstrom von 100 l/h geleitet, um den gelösten Sauerstoff zu entfernen.

Dann wurden 2,50 g einer 0,5 %igen Lösung von Ascorbinsäure in Wasser zugesetzt, homogenisiert und die erhaltene Mischung zwischen zwei Teflonplatten gefüllt, die durch eine Gummidichtung auf 1 mm Abstand gehalten wurden.

Die Teflonplatten wurden in ein 50°C warmes Wasserbad gestellt.

Nach Ablauf der Reaktion wurde eine 1 mm dicker Gelfilm mit einem Wassergehalt von 23 % erhalten. Er war sehr stark klebrig.

Vergleichsbeispiel 5

In einem geschlossenen Kolben mit Rührer und Stickstoffeinleitung von unten wurde aus den folgenden Komponenten eine Mischung hergestellt:

| | |
|---|---|
| 180,00 | Acrylsäure (2,50 mol) |
| 186,50 | Triethanolamin (1,25 mol) |
| 118,50 | Wasser |
| 4,00 | Trimethylolpropantriacrylat |
| 3,33 | einer 15 %igen, wässrigen Natriumperoxodisulfatlösung |

Durch die Mischung wurde für 20 min ein Stickstoffstrom von 100 l/h geleitet um den gelösten Sauerstoff zu entfernen.

Dann wurden 2,50 g einer 0,5 %igen Lösung von Ascorbinsäure in Wasser zugesetzt, homogenisiert und die erhaltene Mischung zwischen zwei Teflonplatten gefüllt, die durch eine Gummidichtung auf 1 mm Abstand gehalten wurden.

Die Teflonplatten wurden in ein 50°C warmes Wasserbad gestellt.

Die erhaltene Gelfolie wurde im Vakummtrockenschrank bei 70°C vollständig getrocknet und anschließend in einer Polyethylentüte mit der notwendigen Wassermenge versetzt um im Gel ein Restfeuchtegehalt von 10 % einzustellen und zugeschweißt. Nach einer Wartezeit von 10 Tagen war die Gelschicht gleichmäßig durchfeuchtet.

Vergleichsbeispiel 6 (entsprechend JP-A-08073507)

In einem geschlossenen Kolben mit Rührer und Stickstoffeinleitung von unten wurde aus den folgenden Komponenten eine Mischung hergestellt:

| | |
|---|---|
| 180,00 | Acrylsäure (2,50 mol) |
| 149,00 | Triethanolamin (1,00 mol) |
| 14,00 | KOH (0,25 mol) |
| 86,00 | Wasser |
| 0,10 | Trimethylolpropantriacrylat |
| 3,33 | einer 15 %igen, wässrigen Natriumperoxodisulfatlösung |

Durch die Mischung wurde für 20 min ein Stickstoffstrom von 100 l/h geleitet um den gelösten Sauerstoff zu entfernen.

Dann wurden 2,50 g einer 0,5 %igen Lösung von Ascorbinsäure in Wasser zugesetzt, homogenisiert und die erhaltene Mischung zwischen zwei Teflonplatten gefüllt, die durch eine Gummidichtung auf 1 mm Abstand gehalten wurden.

Die Teflonplatten wurden in ein 50°C warmes Wasserbad gestellt.

Nach Ablauf der Reaktion wurde eine 1 mm dicker Gelfilm mit einem Wassergehalt von 21 % erhalten. Er

war extrem klebrig.

Tabelle 1: Flexibilität der Schäume

| Beispiel | Amin | Zusammensetzung [mol%] | | | Flexibiltät nach dem Trocknen | Flexibilität nach Klimalagerung bei einer Temperatur/rel. Feuchte von | | | |
| --- | --- | --- | --- | --- | --- | --- | --- | --- | --- |
| | | Ammoni-um–acrylat | Na–Acrylat | Acrylsäure | | 20°C/32% | 20°C/20% | 20°C/15% | 55°C/20% |
| Vergl 1 | – | 0 | 75 | 25 | hart | hart | hart | hart | hart |
| Vergl 2 | – | 0 | 50 | 50 | hart | hart | hart | hart | hart |
| Vergl 3 | – | 0 | 30 | 70 | hart | hart | hart | hart | hart |
| 1 | TEA[a] | 45 | 30 | 25 | hart | flexibel | flexibel | kaum flexi-bel | zähelastisch |
| 2 | TEA[a] | 45 | 0 | 55 | zähelastisch | flexibel | flexibel | zähelastisch | flexibel |
| 3 | TEA[a] | 60 | 15 | 25 | zähelastisch | flexibel | flexibel | flexibel | flexibel |
| 4 | TEA[a] | 60 | 0 | 40 | flexibel | flexibel | flexibel | flexibel | flexibel |
| 5 | TEA[a] | 67.4 | 7.6 | 25 | zähelastisch | flexibel | flexibel | flexibel | flexibel |
| 6 | TEA[a] | 70 | 0 | 30 | flexibel | flexibel | flexibel | flexibel | flexibel |
| 7 | TEA[a] | 75 | 0 | 25 | flexibel | flexibel | flexibel | flexibel | flexibel |
| 8 | MDA[b] | 50 | 25 | 25 | hart | flexibel | flexibel | – | – |

a)   TEA = Triethanolamin
b)   MDA = Methyldiethanoamin

EP 1 059 947 B1

Tabelle 2:  Flexibilität der Schäume bei tiefen Temperaturen

| Beispiel | Amin | Zusammensetzung [mol%] | | | Flexibilität nach Lagerung bei einer Temperatur | | |
|---|---|---|---|---|---|---|---|
| | | Ammonium–acrylat | Na–Acrylat | Acrylsäure | +20°C | +2°C | −15°C |
| Vergl 1 | — | 0 | 75 | 25 | hart | hart | hart |
| Vergl 2 | — | 0 | 50 | 50 | hart | hart | hart |
| Vergl 3 | — | 0 | 30 | 70 | hart | hart | hart |
| 1 | TEA[a] | 45 | 30 | 25 | flexibel | flexibel | flexibel |
| 2 | TEA[a] | 45 | 0 | 55 | flexibel | flexibel | flexibel |
| 3 | TEA[a] | 60 | 15 | 25 | flexibel | flexibel | flexibel |
| 4 | TEA[a] | 60 | 0 | 40 | flexibel | flexibel | flexibel |
| 5 | TEA[a] | 67.4 | 7.6 | 25 | flexibel | flexibel | flexibel |
| 6 | TEA[a] | 70 | 0 | 30 | flexibel | flexibel | flexibel |
| 7 | TEA[a] | 75 | 0 | 25 | flexibel | flexibel | flexibel |
| 8 | MDA[b] | 50 | 25 | 25 | flexibel | flexibel | zähelastisch |

a)  TEA = Triethanolamin

EP 1 059 947 B1

Tabelle 3: Absorptionseigenschaften

| Beispiel | Amin | Zusammensetzung [mol%] | | | AS [g/g sec] | Aufnahme [g/g] | Feuchtege-halt [%] | Aufnahme bei 100% Fest-gehalt [g/g] |
|---|---|---|---|---|---|---|---|---|
| | | Ammoni-um–acrylat | Na–Acrylat oder K–Acrylat | Acrylsäure | | | | |
| Vergl 1 | – | – | 75 | 25 | 0.79 | 36.2 | 25 | 48.3 |
| Vergl 2 | – | – | 50 | 50 | 1.29 | 37.4 | 25 | 49.8 |
| Vergl 3 | – | – | 30 | 70 | 1.84 | 34.1 | 25 | 45.5 |
| Vergl 4 | TEA[a] | 20 | 30 | 50 | <0.02 | 12.5[b]/12.8[c] | 23 | 16.2[b]/16.6[c] |
| Vergl 5 | TEA[a] | 50 | – | 50 | <0.02 | 14.3[b]/24.3[c] | 10 | 18.8[b]/27.0[c] |
| Vergl 6 | TEA[a] | 40 | 10 | 50 | <0.02 | 16.3[b]/35.1[c] | 21 | 20.6[b]/44.4[c] |
| 9 | TEA[a] | 50 | 20 | 30 | 2.91 | 51.7 | 10 | 57.4 |
| 10 | TEA[a] | 48 | 17 | 35 | 3.25 | 55.9 | 10 | 62.1 |
| 11 | TEA[a] | 65 | 10 | 25 | 3.17 | 49.8 | 10 | 55.3 |
| 12 | TEA[a] | 60 | 0 | 40 | 3.07 | 52.3 | 10 | 58.1 |

a)   TEA = Triethanolamin
b)   Die Aufnahmewerte wurden nach einer Quellzeit von 60 min ermittelt
c)   Die Aufnahmewerte wurden nach einer Quellzeit von 18 h ermittelt

EP 1 059 947 B1

**EP 1 059 947 B1**

**Patentansprüche**

1. Wasserabsorbierende, schaumförmige, vernetzte Polymerisate, die erhältlich sind durch

   (I) Schäumen einer polymerisierbaren wäßrigen Mischung, die

   (a) Säuregruppen enthaltende monoethylenisch ungesättigte Monomere, die gegebenenfalls neutralisiert sind,

   (b) gegebenenfalls andere monoethylenisch ungesättigte Monomere,

   (c) Vernetzer,

   (d) Initiatoren,

   (e) 0,1 bis 20 Gew.-% mindestens eines Tensids,

   (f) gegebenenfalls mindestens einen Lösevermittler und

   (g) gegebenenfalls Verdicker, Schaumstabilisatoren, Polymerisationsregler, Füllstoffe und/oder Zellkeimbilder

   enthält, wobei das Schäumen durch Dispergieren von feinen Blasen eines gegenüber Radikalen inerten Gases in die polymerisierbare wäßrige Mischung erfolgt, und

   (II) Polymerisieren der geschäumten Mischung unter Bildung eines schaumförmigen Hydrogels und gegebenenfalls Einstellen des Wassergehalts des schaumförmigen Polymerisats auf 1 bis 60 Gew.-%, **dadurch gekennzeichnet, daß** mindestens 20 Mol-% der Säuregruppen enthaltenden Monomeren (a) mit tertiären Alkanolaminen neutralisiert und/oder daß die freien Säuregruppen des schaumförmigen Hydrogels nach dem Polymerisieren zu mindestens 20 Mol-% mit mindestens einem Alkanolamin neutralisiert worden sind.

2. Wasserabsorbierende, schaumförmige, vernetzte Polymerisate nach Anspruch 1, **dadurch gekennzeichnet, daß** mindestens 40 Mol-% der Säuregruppen enthaltenden Monomeren (a) mit tertiären Alkanolaminen neutralisiert sind.

3. Wasserabsorbierende, schaumförmige, vernetzte Polymerisate nach Anspruch 1, **dadurch gekennzeichnet, daß** die Säuregruppen der schaumförmigen Hydrogele zu mindestens 40 Mol-% mit mindestens einem Alkanolamin neutralisiert sind, wobei die Neutralisation nach Abschluß der Polymerisation erfolgt.

4. Wasserabsorbierende, schaumförmige, vernetzte Polymerisate nach Anspruch 2, **dadurch gekennzeichnet, daß** die Alkanolamine ausgewählt sind aus der Gruppe Triethanolamin, Methyldiethanolamin, Dimethylaminodiglykol, Dimethylethanolamin und N,N,N',N'-Tetra(hydroxyethyl)ethylendiamin.

5. Verfahren zur Herstellung von wasserabsorbierenden, schaumförmigen, vernetzten Polymerisaten, wobei man eine polymerisierbare Mischung aus

   (a) Säuregruppen enthaltenden monoethylenisch ungesättigten Monomeren, die gegebenenfalls neutralisiert sind,

   (b) gegebenenfalls anderen monoethylenisch ungesättigten Monomeren,

   (c) Vernetzer,

   (d) gegebenenfalls mindestens einem Polymerisationsinitiator,

   (e) 0,1 bis 20 Gew.-% mindestens einem Tensid,

   (f) gegebenenfalls mindestens einem Lösevermittler und

(g) gegebenenfalls Verdickern, Schaumstabilisatoren,Polymerisationsreglern, Füllstoffen und/oder Zellkeimbildnern

in einer ersten Verfahrensstufe durch Dispergieren von feinen Blasen eines gegenüber Radikalen inerten Gases schäumt und den so erhaltenen Schaum in einer zweiten Verfahrensstufe unter Bildung eines schaumförmigen Hydrogels polymerisiert und gegebenenfalls den Wassergehalt des schaumförmigen Polymers auf 1 bis 60 Gew.-% einstellt, **dadurch gekennzeichnet, daß** man mindestens 20 Mol-% der Säuregruppen enthaltenden Monomeren (a) mit tertiären Alkanolaminen neutralisiert und/oder daß man die freien Säuregruppen des schaumförmigen Hydrogels nach dem Polymerisieren zu mindestens 20 Mol-% mit mindestens einem Alkanolamin neutralisiert.

6. Verfahren nach Anspruch 5, **dadurch gekennzeichnet, daß** die Alkanolamine ausgewählt sind aus der Gruppe Triethanolamin, Methyldiethanolamin, Dimethylaminodiglykol, Dimethylethanolamin, Ethanolamin und N,N,N',N'-Tetra(hydroxyethyl)ethylendiamin.

7. Verfahren nach Anspruch 5 oder 6, **dadurch gekennzeichnet, daß** man mindestens 40 Mol-% der Säuregruppen enthaltenden Monomeren (a) mit tertiären Alkanolaminen neutralisiert und/oder daß man die freien Säuregruppen des schaumförmigen Hydrogels nach dem Polymerisieren zu mindestens 40 Mol·% mit mindestens einem Alkanolamin neutralisiert.

8. Verwendung der wasserabsorbierenden, schaumförmigen, vernetzten Polymerisate nach den Ansprüchen 1 bis 4 oder erhältlich nach einem Verfahren der Ansprüche 5 bis 7 in Sanitärartikeln, die zur Absorption von Körperflüssigkeiten eingesetzt werden, in Verbandmaterial zur Abdeckung von Wunden, als Dichtungsmaterial, als Bodenverbesserungsmittel, als Bodenersatzstoff und als Verpackungsmaterial.

9. Verwendung der wasserabsorbierenden, schaumförmigen, vernetzten Polymerisate nach den Ansprüchen 1 bis 4 oder erhältlich nach einem Verfahren der Ansprüche 5 bis 7 in Form eines Pulvers mit einem mittleren Teilchendurchmesser von 150 µm bis 5 mm in Sanitärartikeln, die zur Absorption von Körperflüssigkeiten eingesetzt werden, in Verbandmaterial zur Abdeckung von Wunden, als Dichtungsmaterial, als Bodenverbesserungsmittel und als Bodenersatzstoff zum Kultivieren von Pflanzen.

**Claims**

1. A water-absorbing, expanded, crosslinked polymer obtainable by

(I) foaming a polymerizable aqueous mixture which comprises

(a) monoethylenically unsaturated monomers which contain acidic groups and are optionally neutralized,

(b) optionally other monoethylenically unsaturated monomers,

(c) crosslinkers,

(d) initiators,

(e) 0.1-20% by weight of at least one surfactant,

(f) optionally at least one solubilizer and

(g) optionally thickeners, foam stabilizers, polymerization regulators, fillers and/or cell nucleating agents,

where the foaming takes place by dispersing fine bubbles of a gas which is inert to free radicals into the polymerizable aqueous mixture, and

(II) polymerizing the foamed mixture to form an expanded hydrogel and, where appropriate, adjusting the water content of the expanded polymer to 1-60% by weight, if at least 20 mol % of the monomers (a) which contain acidic groups have been neutralized with tertiary alkanolamines and/or the free acidic groups of the expanded hydrogel have been at least 20 mol % neutralized with at least one alkalolamine after the polymerization.

2. A water-absorbing, expanded, crosslinked polymer as claimed in claim 1, wherein at least 40 mol % of the monomers (a) which contain acidic groups are neutralized with tertiary alkanolamines.

3. A water-absorbing, expanded, crosslinked polymer as claimed in claim 1, wherein the acidic groups of the expanded hydrogels are at least 40 mol% neutralized with at least one alkanolamine, the neutralisation taking place after the polymerization.

4. A water-absorbing, expanded, crosslinked polymer as claimed in claim 2, wherein the alkanolamines are selected from the group of triethanolamine, methyldiethanolamine, dimethylaminodiglycol, dimethylethanolamine and N,N,N',N'-tetra(hydroxyethyl)ethylenediamine.

5. A process for producing water-absorbing, expanded, crosslinked polymers, which entails foaming a polymerizable mixture of

(a) monoethylenically unsaturated monomers which contain acidic groups and are optionally neutralized,

(b) optionally other monoethylenically unsaturated monomers,

(c) crosslinkers,

(d) optionally at least one polymerization initiator,

(e) 0.1-20% by weight of at least one surfactant,

(f) optionally at least one solubilizer and

(g) optionally thickeners, foam stabilizers, polymerization regulators, fillers and/or cell nucleating agents,

in a first stage by dispersing fine bubbles of a gas which is inert to free radicals, and polymerizing the resulting foam in a second stage to form an expanded hydrogel, and, where appropriate, adjusting the water content of the expanded polymer to 1-60% by weight, wherein at least 20 mol % of the monomers (a) which contain acidic groups are neutralized with tertiary alkanolamines and/or the free acidic groups of the expanded hydrogel are at least 20 mol % neutralized with at least one alkalolamine after the polymerization.

6. A process as claimed in claim 5, wherein the alkanolamines are selected from the group of triethanolamine, methyldiethanolamine, dimethylaminodiglycol, dimethylethanolamine, ethanolamine and N,N,N',N'-tetra(hydroxyethyl)ethylenediamine.

7. A process as claimed in claim 5 or 6, wherein at least 40 mol % of the monomers (a) which contain acidic groups are neutralized with tertiary alkanolamines and/or the free acidic groups of the expanded hydrogel are at least 40 mol % neutralized with at least one alkalolamine after the polymerization.

8. The use of the water-absorbing, expanded, crosslinked polymers as claimed in claims 1 to 4 or obtainable by a process of claims 5 to 7 in hygiene articles employed to absorb body fluids, in dressing material for covering wounds, as sealing material, as soil improver, as soil substitute and as packaging material.

9. The use of the water-absorbing, expanded, crosslinked polymers as claimed in claims 1 to 4 or obtainable by a process of claims 5 to 7 in the form of a powder with an average particle diameter of 150 $\mu$m to 5 mm in hygiene articles employed to absorb body fluids, in dressing material for covering wounds, as sealing material, as soil improver and as soil substitute for growing plants.

**Revendications**

1. Polymères réticulés hygroscopiques sous forme de mousse que l'on peut obtenir

(I) en faisant mousser un mélange aqueux polymérisable qui contient

(a) des monomères à insaturation monoéthylénique contenant des groupes acides, qui ont éventuellement été neutralisés,
(b) éventuellement d'autres monomères à insaturation monoéthylénique,
(c) des agents réticulants,
(d) des amorceurs,
(e) 0,1% à 20% en poids d'au moins un tensio-actif,
(f) éventuellement au moins un solubilisant et
(g) éventuellement des épaississants, des stabilisants de mousse, des régulateurs de polymérisation, des charges et/ou des agents nucléants,

le moussage étant réalisé en dispersant de fines bulles d'un gaz inerte envers les radicaux libres dans le mélange aqueux polymérisable, et
(II) en polymérisant le mélange expansé en formant un hydrogel sous forme de mousse et éventuellement en ajustant la teneur en eau du polymère sous forme de mousse à une valeur allant de 1% à 60% en poids, **caractérisés en ce qu'**au moins 20% en moles des monomères (a) contenant des groupes acides ont été neutralisés avec des alcanolamines tertiaires et/ou **en ce qu'**au moins 20% en moles des groupes acides libres de l'hydrogel sous forme de mousse ont été neutralisés après la polymérisation avec au moins une alcanolamine.

2. Polymères réticulés hygroscopiques sous forme de mousse selon la revendication 1, **caractérisés en ce qu'**au moins 40% en moles des monomères (a) contenant des groupes acides ont été neutralisés avec des alcanolamines tertiaires.

3. Polymères réticulés hygroscopiques sous forme de mousse selon la revendication 1, **caractérisés en ce qu'**au moins 40% en moles des groupes acides des hydrogels sous forme de mousse ont été neutralisés avec au moins une alcanolamine, la neutralisation ayant été réalisée une fois la polymérisation achevée.

4. Polymères réticulés hygroscopiques sous forme de mousse selon la revendication 2, **caractérisés en ce que** les alcanolamines sont choisies dans le groupe formé par la triéthanolamine, la méthyldiéthanolamine, le diméthylaminodiglycol, la diméthyléthanolamine et la N,N,N',N'-tétra(hydroxyéthyl)éthylènediamine.

5. Procédé de préparation de polymères réticulés hygroscopiques sous forme de mousse, dans lequel on fait mousser, dans une première étape de procédé, un mélange polymérisable composé

(a) de monomères à insaturation monoéthylénique contenant des groupes acides, qui ont éventuellement été neutralisés,
(b) éventuellement d'autres monomères à insaturation monoéthylénique,
(c) d'agents réticulants,
(d) éventuellement au moins un amorceur de polymérisation,
(e) 0,1% à 20% en poids d'au moins un tensio-actif,
(f) éventuellement au moins un solubilisant et
(g) éventuellement des épaississants, des stabilisants de mousse, des régulateurs de polymérisation, des charges et/ou des agents nucléants,

en dispersant de fines bulles d'un gaz inerte envers les radicaux libres et, dans une deuxième étape de procédé, en polymérisant la mousse ainsi obtenue en formant un hydrogel sous forme de mousse et éventuellement en ajustant la teneur en eau du polymère sous forme de mousse à une valeur allant de 1% à 60% en poids, **caractérisé en ce que** l'on neutralise au moins 20% en moles des monomères (a) contenant des groupes acides avec des alcanolamines tertiaires et/ou **en ce que** l'on neutralise après la polymérisation au moins 20% en moles des groupes acides libres de l'hydrogel sous forme de mousse avec au moins une alcanolamine.

6. Procédé selon la revendication 5, **caractérisé en ce que** les alcanolamines sont choisies dans le groupe formé par la triéthanolamine, la méthyldiéthanolamine, le diméthylaminodiglycol, la diméthyléthanolamine, l'éthanolamine et la N,N,N',N'-tétra(hydroxyéthyl)éthylènediamine.

7. Procédé selon la revendication 5 ou 6, **caractérisé en ce que** l'on neutralise au moins 40% en moles des monomères (a) contenant des groupes acides avec des alcanolamines tertiaires et/ou **en ce que** l'on neutralise après la polymérisation au moins 40% en moles des groupes acides libres de l'hydrogel sous forme de mousse avec au moins une alcanolamine.

8. Utilisation des polymères réticulés hygroscopiques sous forme de mousse selon l'une quelconque des revendications 1 à 4 ou pouvant être obtenus selon un procédé des revendications 5 à 7 dans des articles hygiéniques qui sont mis en oeuvre pour absorber des liquides corporels, dans des matériaux de pansement pour le recouvrement de plaies, en tant que matériaux d'étanchéité, en tant qu'agents d'amendement des sols, en tant que substituts de sol et en tant que matériaux d'emballage.

9. Utilisation des polymères réticulés hygroscopiques sous forme de mousse selon l'une quelconque des revendications 1 à 4 ou pouvant être obtenus selon un procédé des revendications 5 à 7 sous la forme d'une poudre ayant une granulométrie moyenne de 150 $\mu$m à 5 mm dans des articles hygiéniques qui sont mis en oeuvre pour absorber des liquides corporels, dans des matériaux de pansement pour le recouvrement de plaies, en tant que matériaux d'étanchéité, en tant qu'agents d'amendement des sols et en tant que substituts de sol pour la culture de plantes.